(19) Europäisches Patentamt
European Patent Office
Office européen des brevets

(11) **EP 4 506 692 A1**

(12) **EUROPEAN PATENT APPLICATION**
published in accordance with Art. 153(4) EPC

(43) Date of publication:
**12.02.2025 Bulletin 2025/07**

(51) International Patent Classification (IPC):
***G01N 33/86*** (2006.01)

(21) Application number: **23780093.3**

(52) Cooperative Patent Classification (CPC):
**G01N 33/86**

(22) Date of filing: **23.03.2023**

(86) International application number:
**PCT/JP2023/011644**

(87) International publication number:
**WO 2023/190083 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.04.2022 JP 2022061912**

(71) Applicant: **Sekisui Medical Co., Ltd.**
**Tokyo 103-0027 (JP)**

(72) Inventors:
• **KAWABE, Toshiki**
**Tokyo 103-0027 (JP)**
• **ODA, Yukio**
**Tokyo 103-0027 (JP)**

(74) Representative: **Wächtershäuser & Hartz**
**Patentanwaltspartnerschaft mbB**
**Weinstraße 8**
**80333 München (DE)**

(54) **COAGULATION TIME EXTENSION FACTOR ESTIMATION METHOD**

(57) Provided is a coagulation time extension factor estimation method, comprising: 1) obtaining Tm(X) and Tn(X); and 2) estimating a coagulation time extension factor for a test blood sample on the basis of Tm(X), wherein Tm(X) represents a measurement point or a time at which a coagulation reaction curve of sample M reaches X% of coagulation reaction end point Em, Tn(X) represents a measurement point or a time at which a coagulation reaction curve of sample N reaches X% of coagulation reaction end point En, Em is a coagulation reaction end point in the coagulation reaction curve of sample M, En is a coagulation reaction end point in the coagulation reaction curve of sample N, the sample S is a test blood sample having an extended coagulation time, the sample N is a normal blood sample, the sample M is a sample mixture of the sample S and the sample N, and X is larger than 0 and 100 or smaller.

## Description

Technical Field

The present invention relates to a coagulation time extension factor estimation method.

Background Art

Blood coagulation tests are tests for diagnosing the coagulability of patients' blood by adding predetermined reagents to blood samples of the patients and measuring blood coagulation times and the like. Typical examples of the blood coagulation time include prothrombin times (PT), activated partial thromboplastin times (APTT), and thrombin times. Anomaly in coagulability of blood causes extension of a coagulation time. Examples of causes of the coagulation time extension include the influence of coagulation inhibitors, decrease in the levels of components involved in coagulation, congenital deficiency of blood coagulation factors, and acquired appearance of autoantibodies which inhibit coagulation reaction.

When a coagulation time is extended in a conventional blood coagulation test, a cross mixing test is generally conducted to determine a coagulation time extension factor unless a sample is suspected of containing heparin. In the cross mixing test, APTT immediately after mixing of a blood sample and a normal sample (immediate reaction) and APTT after incubation at 37°C for 2 hours (delayed reaction) are measured, and whether an APTT extension factor is attributed to a coagulation factor inhibitor (anticoagulant factor), a lupus anticoagulant (LA), or coagulation factor deficiency such as hemophilia is determined on the basis of patterns of the immediate reaction and the delayed reaction. However, the conventional cross mixing test requires a great deal of labor and time as described above.

Patent Literature 1 describes a method for obtaining information on a cause of coagulation time extension, comprising the steps of: obtaining first, second, and third coagulation times for a test blood sample, a normal blood sample, and a sample mixture obtained by mixing them; obtaining a fourth, fifth, and sixth coagulation times for the test blood sample, the normal blood sample, and the sample mixture incubated at a predetermined temperature for 45 minutes to 4 hours; obtaining a first quantification index on the basis of the first, second, and third coagulation times; obtaining a second quantification index on the basis of the fourth, fifth, and sixth coagulation times; and obtaining a value of a ratio or a difference between the value of the first quantification index and the value of the second quantification index, or a combined value of the value of the ratio and the value of the difference, wherein the value is information which suggests whether the test blood sample is suspected of containing a coagulation factor inhibitor. However, this method requires incubating a blood sample for a long time.

Patent Literature 2 describes a method for assessing a blood sample, comprising the steps of: mixing test plasma and normal plasma to prepare a plasma mixture; obtaining a parameter about the differentiation of a coagulation waveform of the plasma mixture; and determining whether the test plasma is suspected of being plasma deficient in coagulation factor or suspected of being plasma containing a lupus anticoagulant (LA) or a coagulation factor inhibitor, on the basis of the obtained value of the parameter.

Citation List

Patent Literature

Patent Literature 1: JP-B-6696963
Patent Literature 2: JP-B-6871673

Summary of Invention

Technical Problem

The present invention provides more rapid and convenient method for estimating a coagulation time extension factor for a blood sample.

Solution to Problem

Specifically, the present invention provides the following.

[1] A coagulation time extension factor estimation method, comprising:

1) obtaining Tm(X) and Tn(X); and
2) estimating a coagulation time extension factor for sample S on the basis of Tm(X),

wherein

Tm(X) represents a measurement point or a time at which a coagulation reaction curve of sample M reaches X% of coagulation reaction end point Em,

Tn(X) represents a measurement point or a time at which a coagulation reaction curve of sample N reaches X% of coagulation reaction end point En,

Em is a coagulation reaction end point in the coagulation reaction curve of sample M,

En is a coagulation reaction end point in the coagulation reaction curve of sample N,

the sample S is a test blood sample having an extended coagulation time,

the sample N is a normal blood sample,

the sample M is a sample mixture of the sample S and the sample N, and

X is larger than 0 and 100 or smaller.

[2] The method according to [1], wherein

the 1) further comprises detecting coagulation reaction end point Es in a coagulation reaction curve of the sample S and obtaining Ts(X) on the basis of Es, wherein

Ts(X) represents a measurement point or a time at which the coagulation reaction curve of the sample S reaches X% of Es.

[3] The method according to [1] or [2], wherein

the 2) comprises the steps of:

(i) calculating one or more of indexes Rj,
wherein

$$j \geq 1,$$

each of the indexes Rj is calculated on the basis of at least one member selected from the group consisting of Ts(X), Tm(X), and Tn(X), and

at least one of the indexes Rj is calculated on the basis of at least Tm(X);

(ii) comparing the indexes Rj with threshold Tj; and

(iii) estimating the coagulation time extension factor for the sample S on the basis of the comparison result, wherein

the coagulation time extension factor is lupus anticoagulant positivity, coagulation factor deficiency, or coagulation factor inhibitor positivity.

[4] The method according to [1] or [2], wherein
the 2) comprises:

calculating at least one index selected from the group consisting of the following R1 to R8:

R1: an index which reflects a ratio between a difference between Tm(X) and Tn(X) and a difference between Ts(X) and Tn(X) for specific X,

R2: an index which represents an amount of change in R1(X) in a specific range of X,

R3: an index which reflects a ratio between an amount of change in Tm(X) and an amount of change in Tn(X) in a specific range of X,

R4: an index which reflects a relative rate of change in T(X) in a specific range of X for the sample S or the sample M,

R5: an index which reflects an amount of change in Tm(X) in a specific range of X,

R6: an index which reflects a difference in Tm(X) between the samples M differing in a mixing ratio of the sample S and the sample N,

R7: an index which reflects a ratio between Tm(X) and Tn(X) for specific X, and

R8: an index which represents an amount of change in R7(X) in a specific range of X; and

estimating the coagulation time extension factor for the sample S using the calculated index.

[5] The method according to [2], wherein
the 2) comprises:

calculating the following index:

$$R1(Xa) = [(Tm(Xa) - Tn(Xa)) / (Ts(Xa) - Tn(Xa))]$$

wherein Xa = from 5 to 100; and
estimating the sample S to have lupus anticoagulant positivity when R1(Xa) is higher than threshold T1, or in other cases, estimating the sample S to have coagulation factor deficiency or coagulation factor inhibitor positivity.

[6] The method according to [2], wherein
the 2) comprises:

calculating the following indexes:

$$R4s(Xx,Xy,Xz) = [(Ts(Xx) - Ts(Xy)) / Ts(Xz)]$$

$$R4m(Xx,Xy,Xz) = [(Tm(Xx) - Tm(Xy)) / Tm(Xz)]$$

wherein Xx = from 5 to 30, Xy = from 10 to 95, and Xz = from 5 to 95, or Xx = from 45 to 70, Xy = from 10 to 45, and Xz = from 5 to 95, and Xx # Xy; and
setting threshold T4s for R4s and threshold T4m for R4m, and estimating the sample S to have lupus anticoagulant positivity when R4s of the sample S is lower than the threshold T4s and R4m is higher than the threshold T4m.

[7] The method according to [5] or [6], wherein
the method comprises:

calculating, for the sample S which has not been estimated to have lupus anticoagulant positivity, the following index:

$$R2(Xb,Xc) = [R1(Xb) - R1(Xc)],$$

wherein Xb = from 5 to 55, Xc = from 10 to 95, and Xb # Xc; and
estimating the sample S to have coagulation factor inhibitor positivity when R2(Xb,Xc) is higher than threshold T2, or estimating the sample S to have coagulation factor deficiency when R2(Xb,Xc) is lower than threshold T2.

[8] The method according to [7], wherein
the method comprises:

calculating, for the sample S estimated to have coagulation factor deficiency, the following index:

$$R3(Xd,Xe) = [Tm(Xd) - Tm(Xe)] / [Tn(Xd) - Tn(Xe)]$$

wherein Xd = from 5 to 90, Xe = from 10 to 95, and Xe - Xd $\geq$ 5; and
estimating the sample S to have coagulation factor inhibitor positivity when R3(Xd,Xe) is lower than threshold T3.

[9] The method according to [7], wherein

the method comprises:

as for the sample S estimated to have coagulation factor deficiency, estimating the sample S to have coagulation factor inhibitor positivity when index R3(Xd,Xe) is lower than threshold T3L; and

as for the sample S estimated to have coagulation factor inhibitor positivity, estimating the sample S to be a coagulation factor inhibitor-positive sample with a high inhibitor titer when R3(Xd,Xe) is higher than threshold T3H, or as for the sample S estimated to have coagulation factor inhibitor positivity, estimating the sample S to be a coagulation factor inhibitor-positive sample with an intermediate inhibitor titer when R3(Xd,Xe) is between thresholds T3L and T3H,

wherein

$$R3(Xd,Xe) \; = \; [Tm(Xd) \; - \; Tm(Xe)] \; / \; [Tn(Xd) \; - \; Tn(Xe)]$$

wherein Xd = from 5 to 90, Xe = from 10 to 95, Xe - Xd $\geq$ 5, and

$$T3L \; < \; T3H.$$

[10] The method according to [7], wherein
the method comprises:

calculating, for the sample S estimated to have coagulation factor inhibitor positivity, the following index:
R1(Xa') = [(Tm(Xa') - Tn(Xa')) / (Ts(Xa') - Tn(Xa'))] wherein Xa' = from 5 to 80; and
estimating the sample S to be a coagulation factor inhibitor-positive sample with a high inhibitor titer when R1(Xa') is higher than threshold T1'.

[11] The method according to [5] or [6], wherein
the method comprises:

calculating, for the sample S which has not been estimated to have lupus anticoagulant positivity, the following index:

$$R5(Xb,Xc) \; = \; [Tm(Xc) \; - \; Tm(Xb)]$$

wherein Xb = from 5 to 35, Xc = 20 to 95, and Xb < Xc; and
estimating the sample S to have coagulation factor deficiency when R5(Xb,Xc) is higher than threshold T5L and lower than threshold T5H (wherein T5L < T5H), or in other cases, estimating the sample S to have coagulation factor inhibitor positivity.

[12] The method according to [11], wherein the method comprises, as for the sample S estimated to have coagulation factor inhibitor positivity, estimating the sample S to be a coagulation factor inhibitor-positive sample with a low inhibitor titer when R5(Xb,Xc) is lower than the threshold T5L, or as for the sample S estimated to have coagulation factor inhibitor positivity, estimating the sample S to be a coagulation factor inhibitor-positive sample with a relatively high inhibitor titer when R5(Xb,Xc) is higher than the threshold T5H.

[13] The method according to [5] or [6], wherein
the method comprises:

calculating, for the sample S which has not been estimated to have lupus anticoagulant positivity, the following indexes:

$$R7(Xb) \; = \; [Tm(Xb) \; / \; Tn(Xb)]$$

$$R7(Xb') \; = \; [Tm(Xb') \; / \; Tn(Xb')]$$

$$R8(Xb,Xb') \; = \; [R7(Xb) \; - \; R7(Xb')]$$

wherein Xb = from 5 to 95, Xb' = from 5 to 95, and Xb # Xb'; and

estimating the sample S to have coagulation factor deficiency when R7(Xb) is lower than threshold T7 and R7(Xb') is lower than threshold T7', and estimating the sample S to have coagulation factor inhibitor positivity when R8(Xb,Xb') is higher than threshold T8.

[14] The method according to [5] or [6], further comprising the following:

obtaining $Tm_A(X)$ and $Tm_B(X)$ for the sample S which has not been estimated to have lupus anticoagulant positivity,

wherein $Tm_A(X)$ represents a measurement point or a time at which a coagulation reaction curve of sample mixture A reaches X% of coagulation reaction end point Em, $Tm_B(X)$ represents a measurement point or a time at which a coagulation reaction curve of sample mixture B reaches X% of coagulation reaction end point Em, the sample mixture A is a sample mixture having a volume ratio of the sample S and the sample N of 9:1, the sample mixture B is a sample mixture having a volume ratio of the sample S and the sample N of 1:9, and X is larger than 0 and 100 or smaller;

calculating the following index:

$$R6(Xb) = [Tm_A(Xb) - Tm_B(Xb)]$$

wherein Xb = from 35 to 95; and

estimating the sample S to have coagulation factor deficiency when R6(Xb) is lower than threshold T6, or estimating the sample S to have coagulation factor inhibitor positivity when R6(Xb) is higher than threshold T6.

[15] The method according to [14], wherein the method comprises, as for the sample S estimated to have coagulation factor inhibitor positivity, estimating the sample S to be a coagulation factor inhibitor-positive sample with a high inhibitor titer when R6(Xb) is higher than threshold T6'.

[16] A program for carrying out a method according to [1] or [2].

[17] An apparatus for carrying out a method according to [1] or [2].

Advantageous Effects of Invention

According to the method of the present invention, a coagulation time extension factor for a blood sample can be estimated without incubating the blood sample for a long time and without calculating a parameter about the differentiation of a coagulation reaction curve, unlike a conventional cross mixing test. The method of the present invention allows for more rapid and convenient estimation of a coagulation time extension factor for a blood sample.

Brief Description of Drawings

Figure 1 illustrates one example of a coagulation reaction curve.

Figure 2 describes T(X). The points in time when a coagulation reaction curve reaches 10%, 50%, and 90% of coagulation reaction end point E are indicated as T(10), T(50), and T(90), respectively.

Figure 3 illustrates coagulation reaction curves and T(X) of different types of test samples. A: LA, B: Def, and C: Inh. The upper diagrams each show a coagulation reaction curve (ordinate: amount of scattered light), and the lower diagrams each show T(X). Each diagram also shows data on a normal sample (dotted line).

Figure 4 illustrates coagulation reaction curves and T(X) of sample mixtures. A, B, and C depict 1:1 sample mixtures of LA, Def, and Inh, respectively, with a normal sample. The upper diagrams each show a coagulation reaction curve (ordinate: amount of scattered light), and the lower diagrams each show T(X). Each diagram also shows data on the normal sample (dotted line).

Figure 5 illustrates A: APTT, B: a difference in APTT between before and after warming of a 1:1 sample mixture with a normal sample, and C: a plot of the difference in APTT between before and after warming of the Inh-derived sample mixture against an inhibitor titer, for different types of test samples. Figure 5A also shows data on the normal sample (NP).

Figure 6 illustrates coagulation reaction curves of test samples and sample mixtures. A: three test samples (LA, Def, and Inh) having the same level of APTT (around 100 seconds), and B: 1:1 sample mixtures of the test samples with a normal sample (LA-M11, Def-M11, and Inh-M11). Each diagram also shows data on the normal sample (NP) .

Figure 7 illustrates Ts(X) and Tm(X) of the samples shown in Figure 6. A: LA and LA-M11, B: Def and Def-M11, and C:Inh and Inh-M11. Each diagram also shows data Tn(X) on the normal sample (NP).

Figure 8 illustrates FT1(X) calculated for sample mixtures. A, B, and C depict data on (test sample:normal sample) 1:9, 1:1, and 9:1 sample mixtures, respectively. In each diagram, the dash-dotted line depicts the LA-derived sample mixture, the dash-double dot line depicts the Def-derived sample mixture, and the solid line depicts the Inh-derived sample mixture.

Figure 9 illustrates [FT1(X)-FT1(95)] calculated for sample mixtures. The drawing is as described in Figure 8.

Figure 10 illustrates A: [FT1(X)-FT1(95)], B: FT2(X), and C: FT3(X,95), calculated for 1:1 sample mixtures. In each diagram, the dash-double dot line depicts the Def-derived sample mixture, and the solid line and the broken line depict the Inh-derived sample mixtures with inhibitor titers of 18 and 5, respectively.

Figure 11 illustrates A: index 1; FT1(95), B: index 2; FT1(10), and C: index 3; [FT1(10)-FT1(95)] for test samples calculated in Example 4.1) as well as D: a plot of index 3 against an inhibitor titer for Inh. 1:1 sample mixtures were used in the calculation of Tm(X).

Figure 12 illustrates A: index 3; FT3(10,95) for Def and Inh, and B: a plot of index 3 against an inhibitor titer for Inh calculated in Example 4.2). 1:1 sample mixtures were used in the calculation of Tm(X).

Figure 13 illustrates A: index 1; FT1(10) and B: index 2; FT3(10,85) for test samples calculated in Example 4.4) as well as C: a plot of index 2 against an inhibitor titer for Inh. 9:1 sample mixtures were used in the calculation of Tm(X).

Figure 14 illustrates A: index 1; FT1(95), B: index 2; FT1(10), C: index 3; [FT1(10)-FT1(95)], and D: index 4; FT3(10,95) for test samples calculated in Example 4.5) as well as E: a plot of index 4 against an inhibitor titer for Inh. 1:9 sample mixtures were used in the calculation of Tm(X).

Figure 15 illustrates A: index 1; FT4s(5, 35,50), B: index 2; FT4m(5, 35,50), and C: index 3; FT5(5,35) for test samples calculated in Example 5.1) as well as D: a plot of index 3 against an inhibitor titer for Inh. 1:1 sample mixtures were used in the calculation of Tm(X).

Figure 16 illustrates A: index 1; FT1(5) of a sample mixture and B: index 2; FT6(95) for test samples calculated in Example 5.2) as well as C: a plot of index 2 against an inhibitor titer for Inh. A 9:1 sample mixture for index 1 and 9:1 and 1:9 sample mixtures for index 2 were used in the calculation of Tm(X).

Figure 17 illustrates A: index 1; FT1(45), B: index 2; FT2(45), C: index 3; FT2(5), and D: index 4; FT2(45)-FT2(5) for test samples calculated in Example 6 as well as E: a plot of index 4 against an inhibitor titer for Inh. 1:1 sample mixtures were used in the calculation of Tm(X) .

Figure 18 illustrates A: Percent Correction (PC) and B: Rosner Index (RI) for test samples calculated in Comparative Example 1) as well as C: a plot of PC and D: a plot of RI against an inhibitor titer for Inh. 1:1 sample mixtures were used in the calculation of Tm(X).

Figure 19 illustrates A: Percent Correction (PC) and B: Rosner Index (RI) for test samples calculated in Comparative Example 2) as well as C: a plot of PC and D: a plot of RI against an inhibitor titer for Inh. 9:1 sample mixtures were used in the calculation of Tm(X).

Figure 20 illustrates A: Percent Correction (PC) and B: Rosner Index (RI) for test samples calculated in Comparative Example 3) as well as C: a plot of PC and D: a plot of RI against an inhibitor titer for Inh. 1:9 sample mixtures were used in the calculation of Tm(X).

Figure 21 illustrates an exemplary flow for estimating a coagulation time extension factor by the method of the present invention.

Figure 22 conceptually illustrates the configuration of an automatic analysis apparatus for performing the coagulation time extension factor estimation method of the present invention.

Description of Embodiments

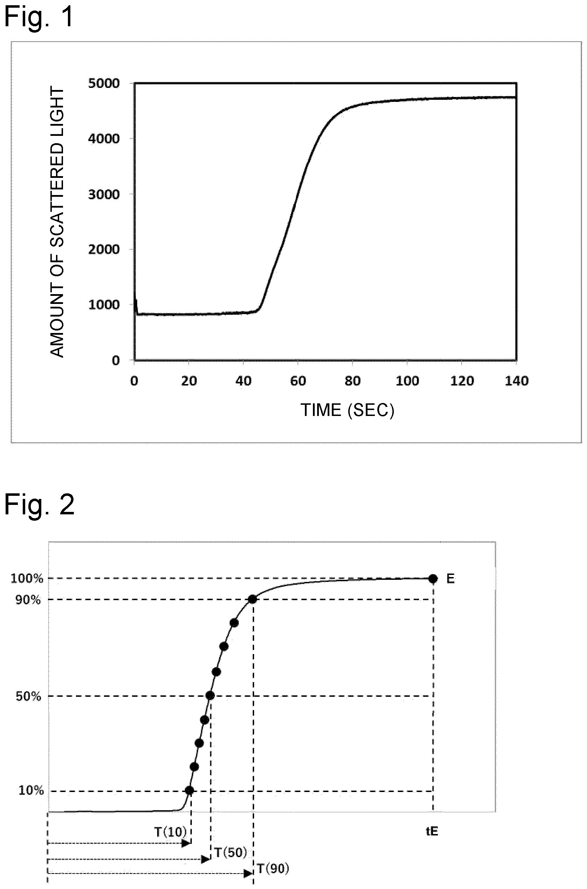

A blood coagulation test involves adding a predetermined reagent to a blood sample, measuring subsequent blood coagulation reaction, and measuring a blood coagulation time from the coagulation reaction. Hereinbelow, the blood sample is also simply referred to as a sample. The measurement of the blood coagulation reaction employs a general approach, for example, an optical approach of measuring an amount of scattered light, a degree of transmission, an absorbance, or the like, or a dynamic approach of measuring a plasma viscosity. The blood coagulation reaction is represented by a coagulation reaction curve which indicates timedependent change in an amount of coagulation reaction. The coagulation reaction curve based on the amount of scattered light of a normal sample having no coagulation anomaly factor usually rises rapidly due to the progression of coagulation after a lapse of a certain time from reagent addition and then reaches a plateau as the coagulation reaction closes to end (see Figure 1). On the other hand, the coagulation reaction curve of an anomal sample having a coagulation anomaly factor exhibits various shapes depending on an anomal factor such as a delayed rise time or a gentle rise in the curve. As a result, in many cases, a coagulation time is extended in the anomal sample compared with the normal sample. The coagulation time extension is an index for the presence or absence of a coagulation anomaly factor. On the other hand, the type of the coagulation anomaly factor (coagulation time extension factor) in the anomal sample cannot be estimated from the coagulation time.

When a coagulation time, for example, APTT, is extended in the blood coagulation test, a cross mixing test is generally conducted to determine a coagulation time extension factor (hereinafter, also simply referred to as an "extension factor") unless a blood sample is suspected of containing heparin. Specifically, whether APTT extension is attributed to a coagulation factor inhibitor (anticoagulant factor), a lupus anticoagulant (LA), or coagulation factor deficiency such as hemophilia is determined. In the cross mixing test, APTT of a normal sample, a test sample, and a sample mixture of the test sample and the normal sample immediately after mixing (immediate reaction) and APTT after incubation at 37°C for 2 hours (delayed reaction) are measured. An APTT extension factor is determined on the basis of patterns of the immediate reaction and the delayed reaction. The conventional general approach described above cannot determine an extension factor unless the cross mixing test is conducted separately from coagulation time measurement. However, the cross mixing test requires measuring the immediate reaction for the sample mixture of the test sample and the normal sample and the delayed reaction after incubation for 2 hours, and is therefore time- and labor-consuming. Specifically, in the conventional cross mixing test, the test sample is determined to have coagulation factor deficiency (Def) or coagulation factor inhibitor positivity (Inh) with a low titer when APTT of the sample mixture is corrected (normalized) with the immediate reaction. On the other hand, whether the test sample has lupus anticoagulant (LA) or Inh cannot be determined when APTT of the sample mixture is not corrected. An extension factor for the test sample is determined by combining these results with results about the delayed reaction. Specifically, if APTT of the sample mixture is not corrected with the immediate reaction, the test sample is determined to have LA or Inh by comparing the results about the delayed reaction with the results about the immediate reaction. On the other hand, if APTT of the sample mixture is corrected with the immediate reaction but not corrected with the delayed reaction, the test sample can be determined to have Inh with a low titer, not Def. As mentioned above, the conventional cross mixing test cannot determine an APTT extension factor unless both the immediate reaction and the delayed reaction are performed. Hence, the cross mixing test cannot meet current clinical needs, for example, needs for determining an extension factor in as short a time as possible after blood collection in an emergency.

Patent Literature 1 mentioned above describes a method for obtaining information on whether or not a blood sample of a test subject contains a coagulation factor inhibitor. However, the method of Patent Literature 1 requires measuring immediate reaction of a normal sample, a test sample, and a sample mixture and delayed reaction after incubation for a long time on the basis of the principles of the cross mixing test and is therefore time- and labor-consuming.

Patent Literature 2 mentioned above proposes a method for obtaining data on an extension factor without incubating a sample for a long time. However, the method of Patent Literature 2 employs a parameter from a differential curve of a coagulation reaction curve, i.e., a coagulation rate (primary differentiation) or a coagulation acceleration (secondary differentiation), in the determination of an extension factor. On the other hand, the coagulation reaction curve, unlike a differential curve, has no peak shape and therefore cannot obtain a parameter based on a peak top, such as a maximum coagulation rate or a maximum coagulation rate time. No attempt has been made so far to extract information on coagulation reaction from the coagulation reaction curve except for a coagulation time.

The present invention enables a coagulation time extension factor for a sample to be estimated by analyzing a waveform of a coagulation reaction curve itself without incubating the sample for a long time (e.g., delayed reaction in a cross mixing test) and without using a differential curve of a coagulation reaction curve (primary differentiation or secondary differentiation), though these procedures are used in the conventional techniques described above.

[Coagulation time extension factor estimation method]

The present invention provides a coagulation time extension factor estimation method. The coagulation time extension factor estimation method of the present invention (hereinafter, also referred to as the method of the present invention) employs a blood sample having an extended coagulation time as a test blood sample (hereinafter, also referred to as a test sample) and estimates an extension factor for the test sample.

A usual blood coagulation test involves obtaining chronological data on blood coagulation reaction of a sample, and obtaining a coagulation reaction curve or a coagulation time for the sample on the basis of the data. A sample having a longer coagulation time than a normal value (e.g., a standard value determined on the basis of a coagulation time of a normal sample group) can be selected as a sample having an extended coagulation time and used as a test sample in the method of the present invention.

Plasma is preferably used as the blood sample for use in the method of the present invention. The sample may be supplemented with an anticoagulant which is usually used in a coagulation test. For example, blood is collected using a blood collection tube containing sodium citrate and then centrifuged to obtain plasma.

The method of the present invention employs the test sample, a normal blood sample (or also simply referred to as a normal sample) which exhibits no coagulation time extension, and a sample mixture obtained by mixing the test sample and the normal sample. Pooled samples having one or more pooled normal sample groups, commercially available normal plasma, or the like can be used as the normal sample. In the preparation of the sample mixture, the test sample and the normal sample are mixed at a predetermined ratio. The mixing ratio of the test sample and the normal sample can be in the range of test sample:normal sample = from 1:9 to 9:1 and is preferably in the range from 4:6 to 6:4, for example, 5:5, in terms of a volume ratio to a total of 10 volumes.

In the method of the present invention, blood coagulation reaction of the test sample, the normal sample, and the sample mixture is measured to obtain coagulation reaction curves. Hereinafter, the test sample is also referred to as sample S, the normal sample is also referred to as sample N, and the sample mixture is also referred to as sample M. The test sample, the normal sample, and the sample mixture are also collectively referred to as a "sample".

Examples of the coagulation time to be measured in the present invention include prothrombin times (PT) and activated partial thromboplastin times (APTT). Hereinbelow, the method of the present invention will be described mainly with reference to the activated partial thromboplastin time (APTT) as the coagulation time. Those skilled in the art are capable of changing or modifying the method of the present invention using another coagulation time (e.g., a prothrombin time (PT)).

Hereinafter, the method of the present invention will be described.

1. Obtainment of coagulation reaction curve

**1.1.** Measurement of blood coagulation reaction

In the measurement of blood coagulation reaction, a coagulation time measurement reagent is added to a sample to start blood coagulation reaction. The coagulation reaction of a reaction solution containing the reagent and the sample may be measured. The coagulation time measurement reagent used can be arbitrarily selected depending on the object of measurement. Various reagents for coagulation time measurement are commercially available (e.g., APTT reagent Coagpia APTT-N; manufactured by Sekisui Medical Co., Ltd.). The measurement of the blood coagulation reaction may employ a general approach, for example, an optical approach of measuring an amount of scattered light, a degree of transmission, an absorbance, or the like, or a dynamic approach of measuring a plasma viscosity. Hereinbelow, the method of the present invention will be described by taking coagulation reaction measurement based on the amount of scattered light as an example.

The start of coagulation reaction may typically be defined as the time when the sample is mixed with the reagent to start coagulation reaction, or may be defined as other timings. The time for which the measurement of coagulation reaction is continued may be, for example, on the order of several tens of seconds to 7 minutes from the mixing of the sample and the reagent. This measurement time may be an arbitrarily set fixed time or may last until the end of coagulation reaction of each sample is detected. Over the measurement time, the progress of coagulation reaction may be repetitively measured (by photometry for optical detection) at predetermined intervals. The measurement can be performed, for example, at 0.1-second intervals. The temperature of the reaction solution during the measurement is a usual condition, for example, 30°C or higher and 40°C or lower, preferably 35°C or higher and 39°C or lower. Various conditions of the measurement may be appropriately set depending on the sample, the reagent, a measurement approach, and the like.

Unlike a conventional cross mixing test or a method described in Patent Literature 1, the method of the present invention does not require further providing a sample subjected to incubation treatment at a predetermined temperature for a long time. The present invention eliminates, for example, the need of subjecting a sample to incubation treatment for a long time, which is performed for delayed reaction in a conventional cross mixing test, before addition of a coagulation time measurement reagent (e.g., a first reagent for APTT measurement) in coagulation reaction measurement. As used herein, the "incubation treatment for a long time" of a sample refers to incubation treatment at preferably 15°C or higher and 40°C or lower, more preferably 30°C or higher and 40°C or lower, for 5 minutes or longer, preferably 2 minutes or longer. As used herein, the "incubation treatment" of a sample refers to active temperature control with a heater, a cooler, a thermostat, or the like and excludes passive change or maintenance of temperature by leaving the sample at room temperature, for example.

On the other hand, in the present invention, the sample to be subjected to coagulation reaction measurement may undergo preliminary warming treatment of the sample for usual coagulation reaction measurement, for example, warming treatment at 30°C or higher and 40°C or lower for 1 minute or shorter, before addition of a coagulation time measurement reagent. Thus, in the present invention, the sample to be subjected to coagulation reaction measurement is not subjected to incubation treatment at 15°C or higher and 40°C or lower for 5 minutes or longer, preferably not subjected to incubation treatment at 15°C or higher and 40°C or lower for 2 minutes or longer, and more preferably, the time of incubation treatment at 15°C or higher and 40°C or lower is 1 minute or shorter, before addition of a coagulation time measurement reagent. In the present invention, the sample to be subjected to coagulation reaction measurement may undergo warming treatment for usual coagulation reaction measurement, i.e., warming treatment so as to keep a reaction solution at 30°C or higher and 40°C or lower, after addition of a coagulation time measurement reagent (after being prepared into a reaction solution).

A series of operations for the coagulation reaction measurement mentioned above can be performed using an automatic analysis apparatus. One example of the automatic analysis apparatus includes automatic blood coagulation analysis apparatus CP3000 (manufactured by Sekisui Medical Co., Ltd.). Alternatively, some of the operations may be manually performed. For example, sample preparation can be performed by a human, and the subsequent operations can be performed in an automatic analysis apparatus.

Coagulation reaction curve P(i) is obtained from the coagulation reaction measurement mentioned above. In this context, "i" represents the number of measurement points or a time from the start of coagulation reaction (also simply referred to as a time). For example, at measurement (photometry) intervals of 0.1 seconds, time = 0.1 × the number of measurement points. In short, P(i) may be a function of the number of measurement points or a function of the time. Hereinbelow, P(i) may be simply abbreviated to P. In general, the coagulation reaction curve P is prepared by subjecting a measurement value of coagulation reaction measurement to noise removal or smoothing treatment, or if necessary, zero point adjustment for adjusting a reaction initial value or obtainment of a relative value of the curve by a known approach. Figure 1 shows one example of the coagulation reaction curve. In Figure 1, the abscissa depicts the time, and the ordinate depicts the amount of scattered light. The coagulation reaction of the reaction solution proceeds with time. Therefore, the amount of scattered light increases and reaches a plateau as the coagulation reaction closes to end.

A differential curve, for example, a reaction rate curve (primary differentiation) or reaction acceleration curve (secondary differentiation), may be obtained, if necessary, from the determined coagulation reaction curve P in order to calculate a coagulation time, for example. The differentiation treatment of the coagulation reaction curve can be carried out by an arbitrary approach and may be performed, for example, by calculating an average slope value within an interval. In the present specification, the primary differential curve of the reaction rate curve and the secondary differential curve of the reaction acceleration curve or the like are also referred to as a "primary curve" and a "secondary curve", respectively.

1.2. Calculation of coagulation time

The blood coagulation time of the sample can be calculated on the basis of the coagulation reaction curve P. The calculation of the coagulation time can be performed in accordance with an arbitrary method. Examples of the coagulation time calculation method include: a method of calculating the point in time when P(i) reaches N% of coagulation reaction end point E (mentioned later) as the coagulation time; a method of calculating the point in time when a primary curve of P reaches N% of its maximum value as the coagulation time; a method of defining the time when the ratio of the integrated value of P(i) in a minute time zone reaches a predetermined value as calculation start point Te, and calculating the point in time when P(i) reaches N % of P(Te) as the coagulation time (JP-A-06-249855); a method of calculating the coagulation time on the basis of timedependent change in the integrated value of P(i) in a minute time zone (see Japanese Patent Application No. 2019-237427); a method of calculating the coagulation time on the basis of the weighted average time of a primary curve of P (see Japanese Patent Application No. 2020-039344); and a method of defining the time when a primary curve of P reaches the maximum value and then reaches a predetermined value as calculation start point Te, and calculating the point in time when P(i) reaches N% of P(Te) as the coagulation time (see Japanese Patent Application No. 2020-068877).

When the coagulation time calculated for a sample derived from a test subject is longer than a normal value (e.g., standard value determined based on the coagulation time of a normal sample group), the sample is determined to have an extended coagulation time. The sample having an extended coagulation time can be used as the test sample in the method of the present invention. When the method of the present invention requires estimating a coagulation time extension factor for the test sample having an extended coagulation time, a sample mixture is prepared from the test sample and coagulation reaction curves of the sample mixture and a normal sample can be obtained.

2. Detection of coagulation reaction end point

The method of the present invention detects coagulation reaction end point E in coagulation reaction curve P of a sample. The coagulation reaction end point E can be detected in accordance with an arbitrary reference such as the point in time when P reaches a plateau, the point in time when a primary curve of P reaches a peak and then decreases to 0 or a given value (see Japanese Patent Application No. 2020-068877), or the earliest point at which the ratio of the integrated value of P(i) in a minute time zone is less than a threshold (e.g., 1.001) (see International Patent Application No. PCT/JP2020/048676 or Example mentioned later). The detection of the coagulation reaction end point E may be performed after obtainment of P until a predetermined measurement time or may be performed simultaneously with obtainment of P and the obtainment of P can be terminated when Pe is detected. For example, procedures described in Japanese Patent Application No. 2020-068877 or International Patent Application No. PCT/JP2020/048676 are capable of detecting the coagulation reaction end point E simultaneously with obtainment of P (detecting E at a so-called real time).

Exemplary procedures of detecting the coagulation reaction end point E based on the method described in International Patent Application No. PCT/JP2020/048676 will be described in detail as one embodiment of the method of the present invention. The ratio of the integrated value of P(i) in a minute time zone is defined as integrated ratio Z(i) and calculated according to the following equation.

$$Z(i) = \{P(i + 1) + P(i + 2) + ... + P(i + m)\} / \{P(i - m) + P(i - m + 1) + ... + P(i - 1)\}$$

In the equation, i represents a measurement point number, and m can be appropriately set depending on coagulation reaction measurement conditions, an analysis item, or the like. For example, at measurement intervals of 0.1 seconds, m = from 10 to 30. P(i) at the earliest measurement point or point in time when Z(i) is less than threshold Zs is detected as the coagulation reaction end point E. Zs may be appropriately set depending on an analysis item and is larger than 1 and 1.100 or less. In the case of, for example, APTT measurement, Zs is preferably 1.050 or less, more preferably in the range from 1.010 to 1.001. For preventing the misdetection of E due to anomal initial reaction or the like, it is preferred to calculate Z(i) when or after i reaches a predetermined calculation start point and after P(i) becomes equal to or larger than a predetermined value. In the present procedures, E can be detected by the obtainment of P(i) and the calculation of Z(i) simultaneously with coagulation reaction measurement.

The method of the present invention can obtain coagulation reaction end point E in a coagulation reaction curve for each of test sample S, normal sample N, and sample mixture M. Hereinafter, the coagulation reaction end point in the coagulation reaction curve of the sample S is referred to as Es, the coagulation reaction end point in the coagulation reaction curve of the sample N is referred to as En, and the coagulation reaction end point in the coagulation reaction curve of the sample M is referred to as Em. In the method of the present invention, preferably, at least Em and En are obtained. More preferably, Es, Em, and En are obtained. As for En, the coagulation reaction of the sample N may be measured and En can be detected from the obtained coagulation reaction curve; En may be detected from the existing coagulation reaction curve of the sample N; or the existing En may be used.

3. Obtainment of T(X)

T(X) which represents a measurement point or a time at which coagulation reaction curve P(i) of a sample reaches X% of E can be calculated for each of the sample S, the sample N, and the sample M. In the present specification, P(i) and T(X) for the sample S are referred to as Ps(i) (or simply Ps) and Ts(X), respectively, P(i) and T(X) for the sample N are referred to as Pn(i) (or simply Pn) and Tn(X), respectively, and P(i) and T(X) for the sample M are referred to as Pm(i) (or simply Pm) and Tm(X), respectively. More specifically, Ts(X) represents a measurement point or a time at which the coagulation reaction curve Ps of the sample S reaches X% of Es, Tn(X) represents a measurement point or a time at which the coagulation reaction curve Pn of the sample N reaches X% of En, and Tm(X) represents a measurement point or a time at which the coagulation reaction curve Pm of the sample M reaches X% of Em. Meanwhile, in the present specification, T(X) is used as a generic name including Ts(X), Tn(X), and Tm(X), and the description about T(X) is also applied to Ts(X), Tn(X), and Tm(X) unless otherwise specified. In the present specification and the drawings, T(X), Ts(X), Tn(X), and Tm(X) are also referred to as "TX", "TsX", "TnX", and "TmX", respectively, without the parentheses of X. In the method of the present invention, preferably, at least Tm(X) and Tn(X) are obtained. More preferably, Ts(X), Tm(X), and Tn(X) are obtained. As for Tn(X), the coagulation reaction of the sample N may be measured and En and Tn(X) can be detected from the obtained coagulation reaction curve; Tn(X) may be detected from the existing coagulation reaction curve of the sample N; or the existing Tn(X) may be used.

More specifically, T(X) is the number of measurement points or a time from the coagulation reaction starting point (time 0) to reaching of P to X% of E. In short, the following equation holds for T(X).

$$P(T(X)) = E \times X\%$$

Thus, the following equations hold for Ts(X), Tn(X), and Tm(X), respectively.

$$Ps(Ts(X)) = Es \times X\%$$

$$Pn(Tn(X)) = En \times X\%$$

$$Pm(Tm(X)) = Em \times X\%$$

In this context, X is larger than 0 and 100 or smaller. Accordingly, T(X) can be represented as a function of the variable X. In the method of the present invention, X is preferably 1 to 100, more preferably 5 to 95. For example, each X may be a value which is 1 or more apart from each other (i.e., an increment of $X \geq 1$) or may be e a value which is 5 or more apart from each other (i.e., an increment of $X \geq 5$). In one example, X is a variable which varies by an increment of 1 in the range from 1 to 100 (i.e., X = {1,2,3,...,97,98,99,100}). In another example, X is a variable which varies by an increment of 5 in the range from 5 to 95 (i.e., X = {5,10,15,...,90,95}).

Alternatively, T(X) may be represented as an average value from T(X - K) to T(X + K). In this case, K is preferably smaller than the increment of X mentioned above. For example, if K = 2, T(X) at X = 10 can be represented as an average value from T(8) to T(12), and T(X) at X = 15 can be represented as an average value from T(13) to T(17). Alternatively, T(X) may be represented as an average value from T(X - K + 1) to T(X). For example, if K = 5, T(X) at X = 10 can be represented as an average value from T(6) to T(10), and T(X) at X = 95 can be represented as an average value from T(91) to T(95).

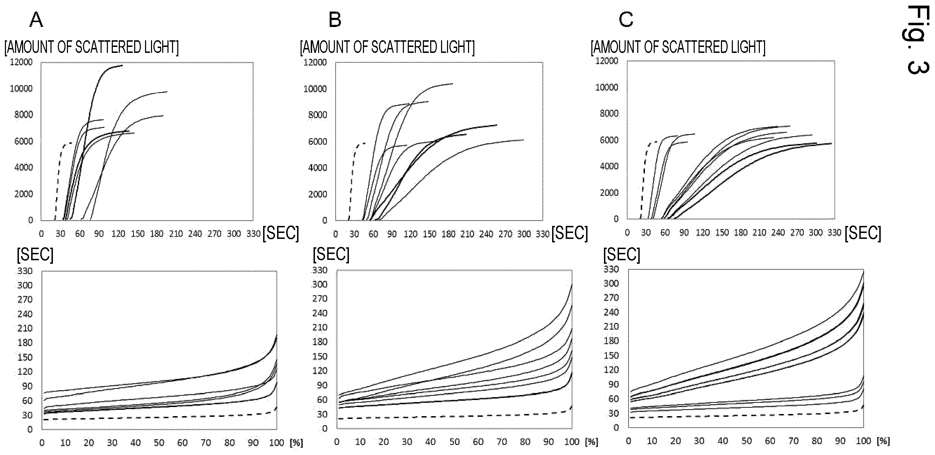

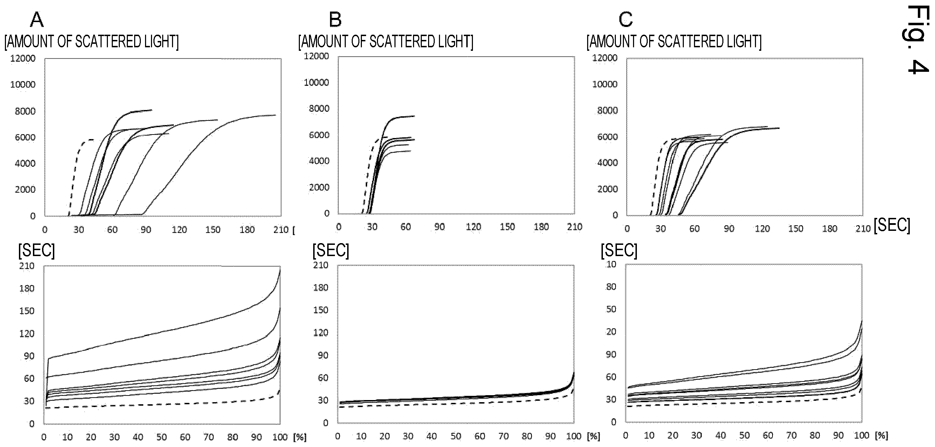

Figure 2 illustrates T(X). The drawing shows a coagulation reaction curve which indicates points at which E reaches 10 to 90%, and the points in time when E reaches 10%, 50%, and 90% are indicated as T(10), T(50), and T(90), respectively. The point in time of E, tE, corresponds to T(100). A T(X) curve obtained by plotting T(X) against X generally rises rapidly, as shown in Figures 3 and 4, from X of 1 to around 5. This curve rises gently up to X of approximately 80 and then rises greatly at or after X on the order of 90.

4. Estimation of coagulation time extension factor using T(X)

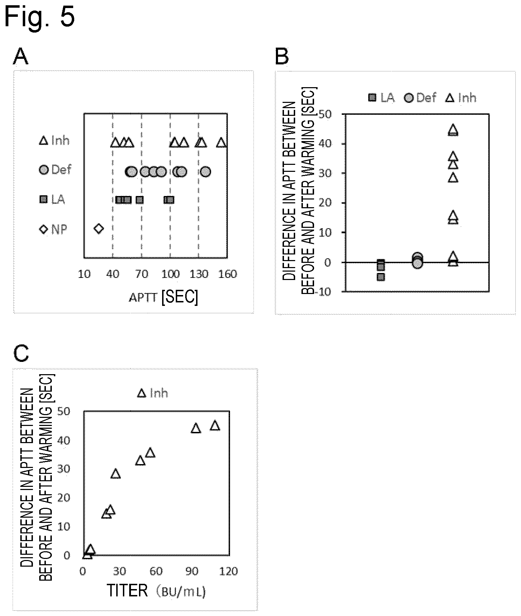

The method of the present invention estimates a coagulation time extension factor for a test sample on the basis of T(X) calculated from a coagulation reaction curve. As shown in Examples mentioned later, the method of the present invention is capable of discriminating, by using T(X), a lupus anticoagulant-positive sample (LA), a coagulation factor-deficient sample (Def), and a coagulation factor inhibitor-positive sample (Inh) from each other without requiring a sample incubated for a long time, which is used for delayed reaction in a cross mixing test, from among test samples which exhibit an extended coagulation time. In conventional general coagulation reaction measurement, only a coagulation time is used as a parameter which is related to the evaluation of coagulation reaction and calculated from a coagulation reaction curve. However, it is difficult to estimate a coagulation time extension factor only from the coagulation time. As shown in Figure 5, samples differing in an extension factor have overlapping coagulation times, and it is still difficult to estimate an extension factor even if the coagulation time is compared between before and after incubation of sample mixture. By contrast, in the present invention, the extension factor is estimated by focusing on the shape itself of a coagulation reaction curve, and calculating T(X) as an index which reflects it.

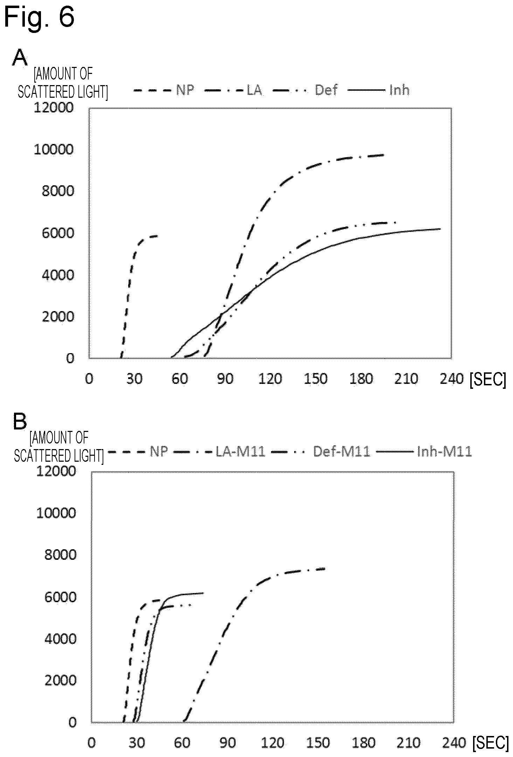

As shown in Figure 6A mentioned later, the coagulation reaction of a sample is capable of exhibiting different features among the types of coagulation time extension factors (sample species). Figure 6A illustrates coagulation reaction curve P (ordinate: the amount of scattered light) of each sample species. In the drawing, all the coagulation times of the test samples (LA, Def, and Inh) are near 100 seconds and are thus extended as compared with a normal sample (NP). The shape of the coagulation reaction curve P exhibits a distinctive tendency depending on a sample species. The curve for the normal sample (NP) rises sharply. The coagulation factor-deficient sample (Def) and the coagulation factor inhibitor-positive sample (Inh) tend to have similar shapes of P, and both of their curves rise relatively gently. The lupus anticoagulant-positive sample (LA) has an extended coagulation time, though its curve rises relatively sharply. As mentioned above, even test samples having the same level of extended APTT differ in the shape of P depending on a sample species.

On the other hand, the coagulation reaction of a sample mixture prepared from a sample having coagulation time extension may exhibit a reaction curve different from that of the original sample. For example, Figure 6B mentioned later illustrates coagulation reaction curve P (ordinate: the amount of scattered light) for sample mixtures (mixing ratio; test sample:NP = 1:1) of the samples shown in Figure 6A. For the sample mixtures (in the drawing, Def-M11 and Inh-M11) derived from the coagulation factor inhibitor-positive sample (Inh) and the coagulation factor-deficient sample (Def), the shape of the coagulation reaction curve P is closer to that of the normal sample (NP) by the replenishment of a coagulation factor included in the normal sample so that the coagulation time falls within a normal range. On the other hand, the sample mixture (LA-M11) derived from the lupus anticoagulant-positive sample (LA) still has an extended coagulation time and is similar in the shape of the coagulation reaction curve P to the original sample (LA in Figure 6A).

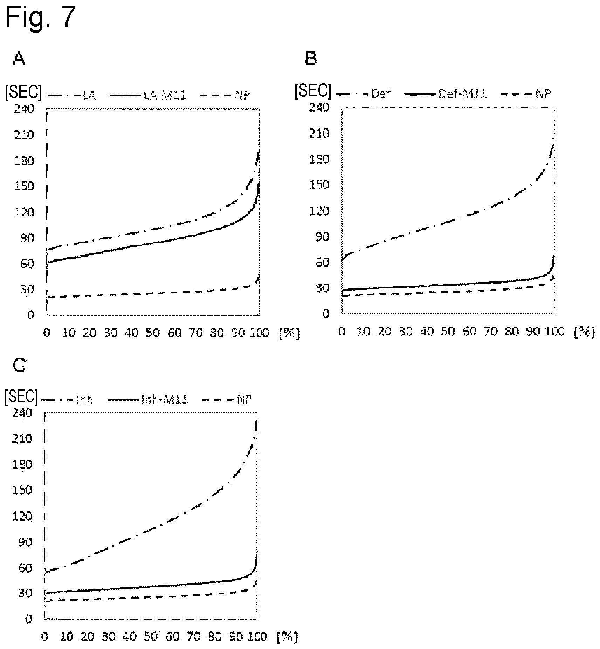

T(X) is also capable of exhibiting different features among sample species. Figures 7A to 7C mentioned later illustrate Ts(X) and Tm(X) on a sample species basis for the test samples respectively shown in Figures 6A and 6B and sample mixtures thereof. Figure 7A depicts the lupus anticoagulant-positive sample (LA) and its sample mixture (LA-M11), Figure 7B depicts the coagulation factor-deficient sample (Def) and its sample mixture (Def-M11), and Figure 7C depicts the coagulation factor inhibitor-positive sample (Inh) and its sample mixture (Inh-M11). Each drawing also shows Tn(X) for the normal sample (NP). In these drawings, APTT (= T(50)) is a value (time) on the ordinate against X = 50 on the abscissa.

As is evident from Figure 7, relative mutual placement of curves which indicate Ts(X), Tm(X), and Tn(X) differs among sample species, mainly due to downward (direction closer to Tn(X)) shift of Tm(X). In short, the downward shift of Tm(X) is small for LA and large for Def and Inh. As a result, the difference between Ts(X) and Tm(X) [Ts(X) - Tm(X)] at arbitrary X is relatively small for LA and relatively large for Def and Inh. Thus, the value of [Ts(X) - Tm(X)] of Def and Inh is larger than that of LA, whereas there is a small difference between [Ts(X) - Tm(X)] of Def and [Ts(X) - Tm(X)] of Inh.

Thus, in the present invention, a coagulation time extension factor for a test sample can be estimated on the basis of at least Tm(X). In one embodiment, the method of the present invention estimates a coagulation time extension factor for a test sample on the basis of the difference between Tn(X) and Tm(X). In one embodiment, the method of the present invention estimates a coagulation time extension factor for a test sample on the basis of the difference among Tm(X), Ts(X), and Tn(X).

4.1. Calculation of index R

In a preferred embodiment, in the method of the present invention, index R for estimating a coagulation time extension factor is calculated using at least Tm(X), and a coagulation time extension factor for a test sample is estimated on the basis of the index R. The index R is an index which is calculated using at least Tm(X) and linked to coagulation reaction information (coagulation characteristics) of the test sample. In one embodiment, the index R is calculated using Tm(X) and Tn(X). In another embodiment, the index R is calculated using Ts(X), Tm(X), and Tn(X). Examples of the index R include R1, R2, R3, R4, R5, R6, R7, R8, and combinations thereof, which will be described below. Thus, as used herein, the "index R" encompasses the case of referring to any one of R1, R2, R3, R4, R5, R6, R7, R8, and the like and the case of referring to a combination of two or more thereof.

In a preferred embodiment, in the method of the present invention, R1 is calculated as an index which reflects a ratio between a difference between Tm(X) and Tn(X) and a difference between Ts(X) and Tn(X) for specific X. In one example, R1 may be represented by the following equation.

$$R1(X) = [(Tm(X) - Tn(X)) / (Ts(X) - Tn(X))]$$

In this context, X is preferably from 5 to 100, more preferably from 5 to 95, further more preferably from 55 to 95. R1(X) may be expressed in percentage.

In a preferred embodiment, in the method of the present invention, R2 is calculated as an index which represents an amount of change in R1 in a specific range of X. Preferably, R2 may be represented by the following equation using $X_1$ and $X_2$.

$$R2(X_1, X_2) = [R1(X_1) - R1(X_2)]$$

In this context, $X_1$ is preferably from 5 to 55, more preferably from 5 to 45, further more preferably from 5 to 40, $X_2$ is preferably from 10 to 95, more preferably from 15 to 95, further more preferably from 20 to 95, and $X_1 \neq X_2$. R2($X_1$,$X_2$) may be expressed in percentage.

In a preferred embodiment, in the method of the present invention, R3 is calculated as an index which reflects a ratio between an amount of change in Tm(X) and an amount of change in Tn(X) in a specific range of X. Preferably, R3 may be represented by the following equation using $X_1$ and $X_2$.

$$R3(X_1, X_2) = [Tm(X_1) - Tm(X_2)] / [Tn(X_1) - Tn(X_2)]$$

In this context, $X_1$ is preferably from 5 to 90, more preferably from 50 to 80, and $X_2$ is preferably from 10 to 95, more preferably from 55 to 85. However, $X_2 - X_1 \geq 5$. R3($X_1$,$X_2$) may be expressed in percentage.

In a preferred embodiment, in the method of the present invention, R4 is calculated as an index which reflects a relative rate of change in T(X) in a specific range of X for the sample S or the sample M. Preferably, R4 may be represented by the following equation using $X_1$, $X_2$, and $X_3$.

$$R4(X_1, X_2, X_3) = [(T(X_2) - T(X_1)) / T(X_3)]$$

More specifically, R4 ($X_1$,$X_2$,$X_3$) can be calculated for each of the sample S and the sample M and may be represented by the following equations, respectively.

$$R4s(X_1, X_2, X_3) = [(Ts(X_2) - Ts(X_1)) / Ts(X_3)]$$

$$R4m(X_1, X_2, X_3) = [(Tm(X_2) - Tm(X_1)) / Tm(X_3)]$$

In each equation, $X_1$ is preferably from 5 to 30, more preferably from 5 to 25, further more preferably from 5 to 20, $X_2$ is preferably from 10 to 95, more preferably from 25 to 95, further more preferably from 25 to 75, and $X_3$ is preferably from 5 to 95, or $X_1$ is preferably from 45 to 70, more preferably from 50 to 65, $X_2$ is preferably from 10 to 45, more preferably from 10 to 35, and $X_3$ is preferably from 5 to 95. $X_1 \neq X_2$, preferably $X_1 < X_2$. R4($X_1$,$X_2$,$X_3$) may be expressed in percentage.

In a preferred embodiment, in the method of the present invention, R5 is calculated as an index which reflects an amount of change in Tm(X) in a specific range of X. Preferably, R5 may be represented by the following equation using $X_1$ and $X_2$.

$$R5(X_1,X_2) = [Tm(X_2) - Tm(X_1)]$$

In the equation, $X_1$ is preferably from 5 to 35, more preferably from 5 to 10, and $X_2$ is preferably from 20 to 95, more preferably from 35 to 65. Preferably, $X_1 < X_2$. $R5(X_1,X_2)$ may be expressed in percentage.

In a preferred embodiment, in the method of the present invention, R6 is calculated as an index which reflects a difference in Tm(X) between the samples M differing in a mixing ratio of the sample S and the sample N. Preferably, R6 may be represented by the following equation.

$$R6(X) = [Tm_A(X) - Tm_B(X)]$$

In this context, $Tm_A(X)$ represents Tm(X) for sample mixture A, and $Tm_B(X)$ represents Tm(X) for sample mixture B. The sample mixtures A and B differ in a test sample:normal sample mixing ratio. The test sample:normal sample mixing ratios of the sample mixtures A and B are not particularly limited. In a preferred embodiment, the sample mixture A has a test sample:normal sample mixing ratio of 9:1, and the sample mixture B has a test sample:normal sample mixing ratio of 1:9. X is preferably from 35 to 95, more preferably from 60 to 95, further more preferably from 90 to 95.

In a preferred embodiment, in the method of the present invention, R7 is calculated as an index which reflects a ratio between Tm(X) and Tn(X) for specific X. Preferably, R7 may be represented by the following equation.

$$R7(X) = [Tm(X) / Tn(X)]$$

In this context, X is preferably from 5 to 95, more preferably from 70 to 95. R7(X) may be expressed in percentage.

In a preferred embodiment, in the method of the present invention, R8 is calculated as an index which represents an amount of change in R7(X) in a specific range of X. In one example, R8 may be represented by the following equation using $X_1$ and $X_2$.

$$R8(X_1,X_2) = [R7(X_1) - R7(X_2)]$$

In this context, $X_1$ is preferably from 10 to 95, more preferably from 15 to 95, further more preferably from 70 to 95, and $X_2$ is preferably from 5 to 90. However, $X_1 - 5 \geq X_2$. R8 (X) may be expressed in percentage.

Each of R1(X), $R2(X_1,X_2)$, $R3(X_1,X_2)$, $R4(X_1,X_2,X_3)$, $R5(X_1,X_2)$, R6(X), R7(X), and $R8(X_1,X_2)$ mentioned above can be calculated using specific one X or one set of $X_1$ to $X_2$ or $X_1$ to $X_3$. Alternatively, a plurality of R1(X), $R2(X_1,X_2)$, $R3(X_1,X_2)$, $R4(X_1,X_2,X_3)$, $R5(X_1,X_2)$, R6(X), R7(X), or $R8(X_1,X_2)$ may be calculated using two or more different X or two or more sets of $X_1$ to $X_2$ or $X_1$ to $X_3$. In this respect, a plurality of values may be calculated for any one, two, or three of R1(X), $R2(X_1,X_2)$, $R3(X_1,X_2)$, $R4(X_1,X_2,X_3)$, $R5(X_1,X_2)$, R6 (X), R7 (X), and R8 $(X_1,X_2)$ described above, and specific one value can be calculated for the remaining members. X, $X_1$ to $X_2$, and $X_1$ to $X_3$. for use in the calculation equations of R1(X), $R2(X_1,X_2)$, $R3(X_1,X_2)$, $R4(X_1,X_2,X_3)$, $R5(X_1,X_2)$, R6(X), R7(X), and $R8(X_1,X_2)$ described above are independent from each other among the equations. For example, X for use in the calculation of R1(X) and X for use in the calculation of R6(X) may be the same value or different values. $X_1$ to $X_2$ for use in the calculation of $R2(X_1,X_2)$ and $X_1$ to $X_2$ for use in the calculation of $R3(X_1,X_2)$ may be the same value or different values.

In one example, R1(Xa) and R2(Xb,Xc) can be calculated using Xa, Xb, and Xc (wherein Xa, Xb, and Xc are values which are the same as or different from each other on the proviso that Xb # Xc).

In another example, R1(Xa) and R3(Xd,Xe) can be calculated using Xa, Xd, and Xe (wherein Xa, Xd, and Xe are values which are the same as or different from each other on the proviso that Xd # Xe).

In an alternative example, R1(Xa) and at least one member selected from the group consisting of R1(Xa'), R2(Xb,Xc), and R3(Xd,Xe) can be calculated using Xa, Xa', Xb, Xc, Xd, and Xe (wherein Xa, Xa', Xb, Xc, Xd, and Xe are values which are the same as or different from each other on the proviso that Xa # Xa', Xb # Xc, and Xd # Xe). In one example, R1(Xa), R2(Xb,Xc), and R3(Xd,Xe) are calculated. In another example, R1(Xa) and R3(Xd,Xe) are calculated. In an alternative example, R1(Xa), R1(Xa'), R2(Xb,Xc), and R3(Xd,Xe) are calculated. In this context, Xa # Xa', Xa is preferably from 5 to 100, more preferably from 5 to 95, further more preferably from 55 to 95, and Xa' is preferably from 5 to 100, more preferably from 5 to 80. Xb and Xc, and Xd and Xe can be selected from the ranges of $X_1$ and $X_2$ regarding R2 $(X_1,X_2)$ and the ranges of $X_1$ and $X_2$ regarding $R3(X_1,X_2)$, respectively, as mentioned above.

In an alternative example, R1(Xa) are R1(Xa') are calculated using Xa and Xa' (wherein Xa < Xa'), and R2(Xa,Xa') can be calculated as a difference therebetween. In this context, Xa and Xa' are preferably from 5 to 55 and from 10 to 95, respectively, more preferably from 5 to 45 and from 15 to 95, respectively, further more preferably from 5 to 40 and from 20 to 95, respectively.

In an alternative example, R4s(Xx,Xy,Xz) and R4m(Xx,Xy,Xz) are calculated using Xx, Xy, and Xz (wherein Xx, Xy, and Xz are values which are the same as or different from each other on the proviso that Xx # Xy), and R5(Xd,Xe) (wherein Xd # Xe) can be further calculated. In this context, each of Xx to Xz can be selected from the ranges of $X_1$ to $X_3$. mentioned above regarding R4($X_1,X_2,X_3$) mentioned above. Each of Xd to Xe can be selected from the ranges of $X_1$ and $X_2$ regarding R5($X_1,X_2$) mentioned above.

In an alternative example, R1(Xa), R6(Xb), and R6(Xb') can be calculated using Xa, Xb, and Xb' (wherein Xa, Xb, and Xb' are values which are the same as or different from each other on the proviso that Xb # Xb'). In this context, Xa can be selected from the range of X regarding R1(X) mentioned above, and each of Xb and Xb' can be selected from the range of X regarding R6(X) mentioned above.

In an alternative example, R1(Xa), R7 (Xb), R7(Xb'), and R8(Xb,Xb') can be calculated using Xa, Xb, and Xb' (wherein Xa, Xb, and Xb' are values which are the same as or different from each other on the proviso that Xb # Xb'). Xa is preferably from 5 to 100, more preferably from 5 to 95, further more preferably from 55 to 95, Xb is preferably from 10 to 95, more preferably from 15 to 95, further more preferably from 70 to 95, and Xb' $\leq$ Xb - 5.

In the example described above, R4s(Xx,Xy,Xz) and R4m(Xx,Xy,Xz) may be calculated instead of calculating R1(Xa). Alternatively, R1(Xa) may be calculated instead of calculating R4s(Xx,Xy,Xz) and R4m(Xx,Xy,Xz).

4.2. Estimation of coagulation time extension factor

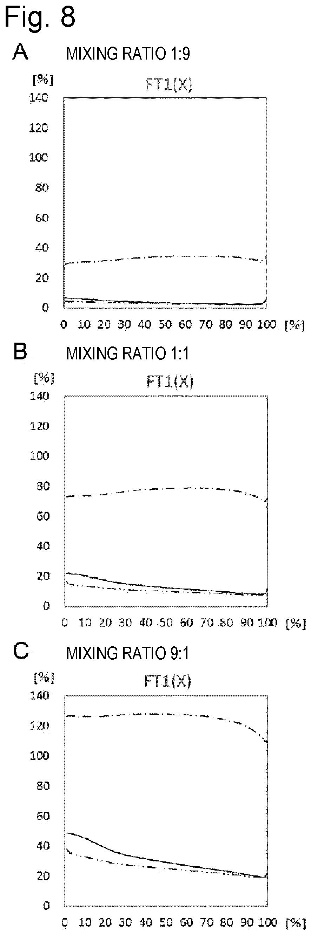

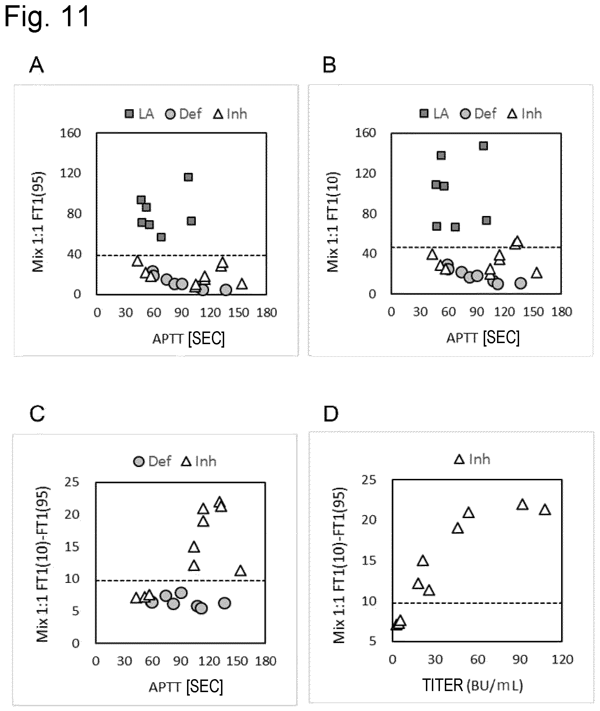

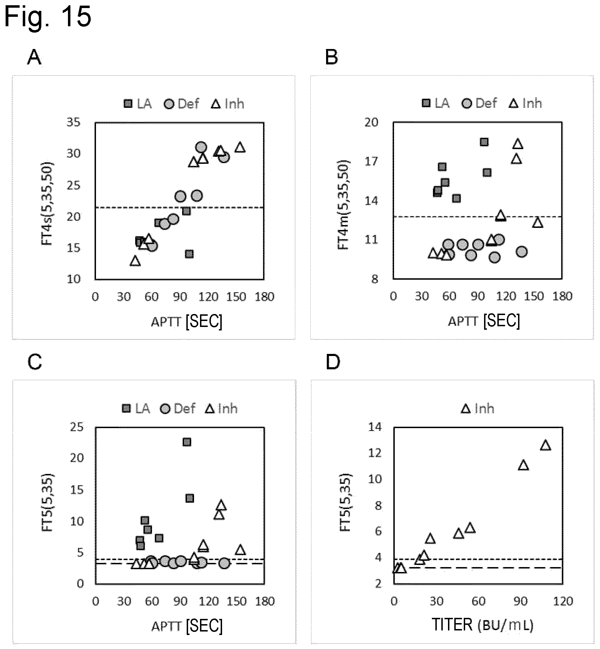

The calculated index R is capable of exhibiting different distributions among the types of coagulation time extension factors (sample species) (see Figures 8 to 10). For example, as shown in Examples mentioned later, R1(X) for lupus anticoagulant positivity (LA) tends to be larger than that for coagulation factor deficiency (Def) and coagulation factor inhibitor positivity (Inh), and R2 ($X_1,X_2$) and R3($X_1,X_2$) for Inh having an inhibitor titer of 18 BU/mL or more tend to be larger than those for Def (see Figures 11 to 14). As for Tm(X), R4($X_1,X_2,X_3$), R5($X_1,X_2$), R6(X), R7(X), and R8($X_1,X_2$) for LA and Inh (except for Inh having a low titer) tend to be larger than those for Def. And R4($X_1,X_2,X_3$) of Ts(X) for Def and Inh bears a proportionate relationship to APTT (see Figures 15 to 17). Thus, the index R can be used for estimating a coagulation time extension factor for a test sample.

In a preferred embodiment, a coagulation time extension factor for a test sample can be estimated on the basis of R1(X), R2($X_1,X_2$), R3($X_1,X_2$), R4($X_1,X_2,X_3$), R5($X_1,X_2$), R6(X), R7(X), R8($X_1,X_2$), or a combination of two or more thereof mentioned above. More specifically, one or more R1(X), one or more R2($X_1,X_2$), one or more R3($X_1,X_2$), one or more R4($X_1,X_2,X_3$), one or more R5($X_1,X_2$), one or more R6(X), one or more R7(X), or one or more R8($X_1,X_2$), or a combination thereof is calculated using one or more X or one or more sets of $X_1$ and $X_2$ or $X_1$ to $X_3$, and a coagulation time extension factor for a test sample can be estimated on the basis of any one thereof, preferably a combination of any two or more thereof.

Examples of the coagulation time extension factor (sample species) which may be estimated by the method of the present invention include lupus anticoagulant positivity (LA), coagulation factor deficiency (Def), and coagulation factor inhibitor positivity (Inh). Examples of the coagulation factor deficiency (Def) include coagulation factor V (FV) deficiency, coagulation factor VIII (FVIII) deficiency, coagulation factor IX (FIX) deficiency, coagulation factor X (FX) deficiency, coagulation factor XI (FXI) deficiency, and coagulation factor XII (FXII) deficiency with FVIII deficiency being preferred. Preferably, the FVIII deficiency refers to a condition having a FVIII activity value of within approximately 5%. Examples of the coagulation factor inhibitor include FVIII inhibitors and FV inhibitors. Preferably, the FVIII inhibitor positivity refers to a state having an inhibitor titer of 3 BU/mL or more, preferably 5 BU/mL or more. In the present specification, the inhibitor titer is also simply referred to as a "titer". In the present specification, the inhibitor titer is indicated in Bethesda unit (BU/mL) .

In one example, a coagulation time extension factor for a test sample is estimated on the basis of R1(Xa). In another example, a coagulation time extension factor for a test sample is estimated on the basis of a combination of R1(Xa) and R2(Xb,Xc) or R3(Xd,Xe) (wherein Xa, Xb, Xc, Xd, and Xe are values which are the same as or different from each other on the proviso that Xb # Xc and Xd # Xe).

In an alternative example, a coagulation time extension factor for a test sample is estimated on the basis of a combination of R1(Xa), R2(Xb, Xc), and R3(Xd,Xe) (wherein Xa, Xb, Xc, Xd, and Xe are values which are the same as or different from each other on the proviso that Xb # Xc and Xd # Xe).

In an alternative example, a coagulation time extension factor for a test sample is estimated on the basis of a combination of R1(Xa), R1(Xa'), and R2(Xb,Xc) (wherein Xa, Xa', Xb, and Xc are values which are the same as or different from each other on the proviso that Xa # Xa' and Xb # Xc).

In an alternative example, a coagulation time extension factor for a test sample is estimated on the basis of a combination of R1(Xa), R1(Xa'), and R2(Xa',Xa) (Xa $\neq$ Xa').

In an alternative example, a coagulation time extension factor for a test sample is estimated on the basis of a combination of R1(Xa), R1(Xa'), R2(Xb,Xc), and R3(Xd,Xe) (wherein Xa, Xa', Xb, Xc, Xd, and Xe are values which are the same as or different from each other on the proviso that Xa # Xa', Xb # Xc, and Xd # Xe).

In an alternative example, a coagulation time extension factor for a test sample is estimated on the basis of a combination of R1(Xa), R1(Xa'), R2(Xa',Xa), and R3(Xd,Xe) (wherein Xa, Xa', Xd, and Xe are values which are the same as or different from each other on the proviso that Xa # Xa' and Xd # Xe).

In an alternative example, a coagulation time extension factor for a test sample is estimated on the basis of a combination of R4s(Xa,Xb,Xc), R4m(Xa,Xb,Xc), and R5 (Xd, Xe) (wherein Xa to Xe are values which are the same as or different from each other on the proviso that Xa # Xb and Xd # Xe, preferably Xa = Xd and Xb = Xe).

In an alternative example, a coagulation time extension factor for a test sample is estimated on the basis of a combination of R1(Xa), R6(Xb), and R6(Xb') (wherein Xa, Xb, and Xb' are values which are the same as or different from each other on the proviso that Xb # Xb').

In an alternative example, a coagulation time extension factor for a test sample is estimated on the basis of a combination of R1(Xa), R7(Xb), R7(Xb'), and R8(Xb,Xb') (wherein Xa, Xb, and Xb' are values which are the same as or different from each other on the proviso that Xb # Xb').

Alternatively, in the method of the present invention, a coagulation time extension factor for a test sample can be estimated by combining the index R (e.g., any of R1 to R8 or a combination thereof) with a known index for use in coagulation time extension factor estimation, for example, Percent Correction (PC) or Rosner Index (RI) mentioned later, or an index using T(X) instead of APTT in the calculation equation of PC or RI.

In one embodiment, the index R calculated from a test sample is compared with estimation threshold (hereinafter, also simply referred to as threshold) T to estimate a coagulation time extension factor for the test sample. The threshold T can be determined prior to the implementation of the method of the present invention. The threshold T can be determined on the basis of the index R calculated from a sample population having a known coagulation time extension factor. The threshold T may be set for each index for use in coagulation time extension factor estimation. For example, thresholds T1 to T8 may be set for R1 to R8, respectively, mentioned above. Alternatively, two or more thresholds T may be set for one index.

In one example, index R of each sample is calculated from a sample population having a known sample species (e.g., LA, Def, or Inh), and threshold T is set on the basis of their statistic (e.g., mean $\pm$ [k $\times$ standard deviation], minimum value / k, or maximum value $\times$ k; k $\geq$ 1). A sample species of a test sample is estimated on the basis of whether or not the index R of the test sample exceeds the threshold T. Exemplary procedures of estimation will be described below in detail.

Procedure 1): R1(Xa) for LA tends to be larger than that for the other sample species (Def and Inh). Accordingly, threshold T1 which can discriminate R1(Xa) for LA from R1(Xa) for Def and Inh can be set and compared with R1(Xa) of a test sample to estimate whether the sample species of the test sample is LA or is Def or Inh. Xa is preferably from 5 to 100, more preferably from 5 to 95, further more preferably from 55 to 95. For example, the test sample is estimated to have LA when R1(Xa) of the test sample is higher than the threshold T1. The test sample is estimated to have Def or Inh when R1(Xa) of the test sample is lower than the threshold T1. In this respect, preferred examples of the threshold T1 include statistics of R1(Xa) obtained from a sample population of Def, and statistics of R1(Xa) obtained from a sample population of LA.

Procedure 2): The test sample estimated to have Def or Inh on the basis of R1(Xa) in the procedure 1) can be further estimated to have Def or Inh using another index.

In one example, a statistic of R2(Xb,Xc) obtained from a sample population of Def can be used as threshold T2. Xb is preferably from 5 to 55, more preferably from 5 to 45, further more preferably from 5 to 40, Xc is preferably from 10 to 95, more preferably from 15 to 95, further more preferably from 20 to 95, and Xb < Xc. The test sample is estimated to have Inh when R2(Xb,Xc) of the test sample is higher than the threshold T2. The test sample is estimated to have Def when R2(Xb,Xc) of the test sample is lower than the threshold T2.

A sample species can be estimated in more detail by further combining the threshold T2 with another threshold. For example, an Inh sample can be further classified depending on an inhibitor titer.

Procedure 2A): An Inh sample with a low inhibitor titer is difficult to discriminate from Def by the threshold T2. In this procedure, a statistic of R3(Xd,Xe) obtained from a sample population of Def is used as threshold T3 and combined with the threshold T2. Xd is preferably from 5 to 90, more preferably from 50 to 80, and Xe is preferably from 10 to 95, more preferably from 55 to 85, on the proviso that Xe - Xd $\geq$ 5. Among the samples estimated to have Def on the basis of the threshold T2 in the procedure 2), a sample with R3(Xd,Xe) lower than the threshold T3 is re-estimated to have Inh. The Inh sample thus re-estimated has Inh having a low inhibitor titer (Inh-L). This procedure enables low-titer Inh having an inhibitor titer of 5 or less to be estimated and is therefore clinically meaningful.

Procedure 2B): Inh is classified in stages depending on an inhibitor titer. Two thresholds T3L and T3H (T3L < T3H) are set on the basis of a statistic of R3(Xd,Xe) obtained from a sample population of Def, and used in combination with the threshold T2. In this context, Xd is preferably from 5 to 90, more preferably from 50 to 80, and Xe is preferably from 10 to 95, more preferably from 55 to 85, on the proviso that Xe - Xd $\geq$ 5.

In this procedure, among the samples estimated to have Def on the basis of the threshold T2 in the procedure 2), a sample with R3(Xd,Xe) lower than the threshold T3L is re-estimated to have Inh, as in the procedure 2A). Such an Inh sample has Inh having a low inhibitor titer (Inh-L). On the other hand, among the samples estimated to have Inh on the basis of the threshold T2 in the procedure 2), a sample with R3(Xd,Xe) higher than the threshold T3H is estimated to have Inh having a high inhibitor titer (Inh-H). The remaining Inh samples with R3(Xd,Xe) between the thresholds T3L and T3H are estimated to have Inh having an intermediate inhibitor titer (Inh-M). Inh-H can be further applied to procedure 2C) mentioned later to estimate Inh having a higher inhibitor titer (Inh-VH).

Procedure 2C): Inh having a higher inhibitor titer can also be classified. In this procedure, second threshold T1' is set for R1(Xa). The threshold T1' is, for example, the statistic from a sample population of Def for R1(Xa) used in the procedure 1) and is a value lower than the threshold T1, or is a statistic from a sample population of Def for R1(Xa'). In this context, Xa > Xa', Xa is preferably from 5 to 100, more preferably from 5 to 95, further more preferably from 55 to 95, and Xa' is preferably from 5 to 80, more preferably from 5 to 50.

For example, threshold T1' is set using R1(Xa') when R1(Xa) is used in the procedure 1). Among the test samples estimated to have Inh in the procedure 2), a sample with R1(Xa') higher than the threshold T1' can be estimated to have Inh having a higher inhibitor titer (e.g., a titer of 70 or more, preferably 80 or more, more preferably 90 or more) (Inh-VH).

Procedure 3): R4s(Xx,Xy,Xz) (wherein Xx is preferably from 5 to 30, more preferably from 5 to 25, further more preferably from 5 to 20, Xy is preferably from 10 to 95, more preferably from 25 to 95, further more preferably from 25 to 75, and Xz is preferably from 5 to 95, or Xx is preferably from 45 to 70, more preferably from 50 to 65, Xy is preferably from 10 to 45, more preferably from 10 to 35, and Xz is preferably from 5 to 95, and Xx # Xy, preferably Xx < Xy) (hereinafter, simply referred to as R4s for simplification) varies depending on APTT, whereas R4s for LA tends to be not a relatively large value even if APTT is extended. On the other hand, R4m(Xx,Xy,Xz) (wherein Xx, Xy, and Xz are as described above) (hereinafter, simply referred to as R4m for simplification) for LA tends to be larger than that for the other sample species (Def and Inh). Accordingly, threshold T4s for R4s and threshold T4m for R4m are set. The test sample can be estimated to have LA when R4s of the test sample is lower than T4s and R4m is higher than T4m. This procedure can be used instead of the procedure 1) mentioned above in the estimation of LA, preferably the discrimination of LA from Def or Inh.

Procedure 3A): Two thresholds T5L and T5H (T5L < T5H) are set for R5(Xb,Xc) (wherein Xb is preferably from 5 to 35, more preferably from 5 to 10, Xc is preferably from 20 to 95, more preferably from 35 to 65, and preferably Xb < Xc) (hereinafter, simply referred to as R5 for simplification) for a test sample which has not been estimated to have LA in the procedure 1) or the procedure 3). A test sample with R5 higher than T5L and lower than T5H can be estimated to have Def, and other test samples can be estimated to have Inh. Among the test samples estimated to have Inh, a sample with R5 lower than T5L mentioned above is further estimated to have Inh having a low inhibitor titer (Inh-L). On the other hand, the remaining samples with R5 higher than T5H are estimated to have Inh having a relatively high inhibitor titer. Among them, a sample with R4m higher than T4m mentioned above is estimated to have Inh having a high inhibitor titer (Inh-H), while a sample with R4m lower therethan is estimated to have Inh having an intermediate inhibitor titer (Inh-M).

Procedure 4): Alternatively, R6(Xb) is determined for a test sample which has not been estimated to have LA in the procedure 1) or the procedure 3). In this context, Xb is preferably from 35 to 95, more preferably from 60 to 95, further more preferably from 90 to 95. A statistic of R6(Xb) obtained from a sample population of Def can be used as each of thresholds T6 and T6'. The test sample is estimated to have Def when R6(Xb) of the test sample is lower than T6. The test sample is estimated to have Inh when R6(Xb) of the test sample is higher than T6. The test sample estimated to have Inh is re-estimated to have Inh having a high inhibitor titer (Inh-H) when R6(Xb) of the test sample is higher than T6'.

Procedure 5): Alternatively, R7 (Xb), R7(Xb'), and R8(Xb,Xb') are determined for a test sample which has not been estimated to have LA in the procedure 1) or the procedure 3). In this context, each of Xb and Xb' is preferably from 5 to 95, more preferably from 70 to 95, on the proviso that Xb # Xb'. More preferably, Xb is preferably from 10 to 95, more preferably from 15 to 95, further more preferably from 70 to 95, Xb' is preferably from 5 to 90, and Xb - 5 ≥ Xb'. Statistics of R7 (Xb), R7(Xb'), and R8(Xb,Xb') obtained from a sample population of Def can be used as thresholds T7, T7' and T8, respectively. The test sample is estimated to have Def when R7(Xb) and R7(Xb') of the test sample are lower than T7 and T7', respectively. The test sample is estimated to have Inh when R8(Xb,Xb') of the test sample is higher than T8.

Procedure 6): LA can be discriminated from the other sample species by determining R1(Xa) and R3(Xd,Xe). Xa is preferably from 5 to 100, more preferably from 5 to 95, further more preferably from 55 to 95. Xd is preferably from 5 to 90, more preferably from 50 to 80, and Xe is preferably from 10 to 95, more preferably from 55 to 85, on the proviso that Xe - Xd ≥ 5. The test sample can be estimated to have LA when R1(Xa) of the test sample is higher than threshold T1 and R3(Xd,Xe) is higher than threshold T3. In this respect, preferred examples of the thresholds T1 and T3 include statistics of R1(Xa) and R3(Xd,Xe) obtained from a sample population of Def, and statistics of R1(Xa) and R3(Xd,Xe) obtained from a sample population of LA.

This procedure can be used in place of the procedure 1) or the procedure 3) mentioned above in the estimation of LA, preferably the discrimination of LA from Def or Inh. It can thus be understood that a test sample which has not been estimated to have LA in this procedure can be subsequently subjected to the procedure 2), the procedures 2A) to 2C), the procedure 3A), the procedure 4), and the procedure 5) mentioned above.

Those skilled in the art can understood that in the procedure 1), the procedure 2), the procedures 2A) to 2C), the procedure 3), the procedure 3A), the procedure 4), the procedure 5), and the procedure 6), "equal to or higher than threshold T1" may be used instead of "higher than threshold T1", or "equal to or lower than threshold T1" may be used instead of "lower than threshold T1", as long as different sample species can be discriminated and estimated. The same holds true for thresholds T1', T2, T3, T3L, T3H, T4s, T4m, T5L, T5H, T6, T6', T7, T7', and T8.

In one embodiment, Percent Correction (PC) or Rosner Index (RI) can be used instead of the procedure 1) using R1(X) or the procedure 3) using R4s and R4m mentioned above to estimate whether the sample species of the test sample is LA or is Def or Inh. PC and RI can be calculated according to the following equations.

Percent Correction (PC) (%) = [(S - M) / (S - N)] $\times$ 100

$$\text{Rosner Index (RI) (\%) = [(M - N) / S]} \times 100$$

(N: APTT of sample N, M: APTT of sample M, and S: APTT of sample S)

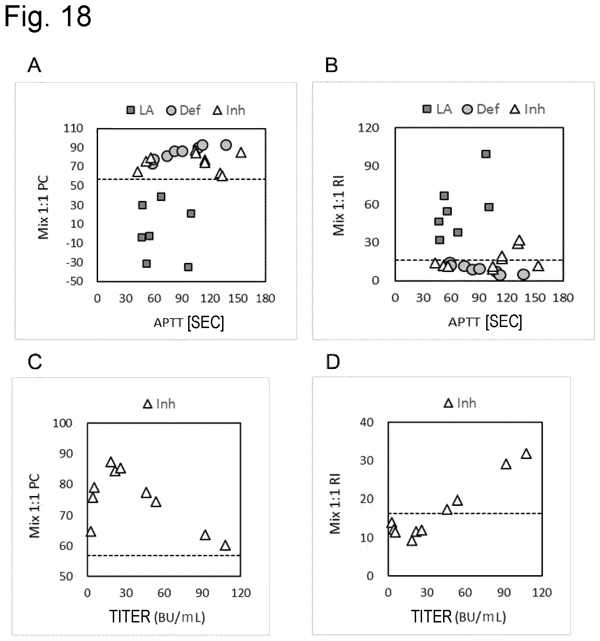

PC for LA is smaller than that for Def and Inh, and RI for LA is larger than that for Def and Inh (see Figures 18 to 20). Preferably, a statistic is calculated for PC or RI from a sample population of LA or Def and used as a threshold of PC or **RI.** The test sample is estimated to have LA when PC of the test sample is equal to or lower than (or lower than) the threshold. Alternatively, the test sample is estimated to have LA when RI of the test sample is equal to or higher than (or higher than) the threshold. Alternatively, the same estimation as above can be performed using arbitrary Ts(X), Tm(X), and Tn(X) instead of APTT of the samples **S, M,** and **N,** respectively, in the equation of PC or RI described above.

The results of estimating the extension factor (sample species) for the test sample can be output in an arbitrary format. Information such as the coagulation time, the coagulation reaction curve, the coagulation reaction end point, and the T(X) curve for the test sample can be output, if necessary, together with the results of estimating the sample species for the test sample. The same information as above may be output for a sample mixture. Preferably, the results of estimating the sample species for the test sample and the coagulation time of the test sample are both output.

5. Estimation flow

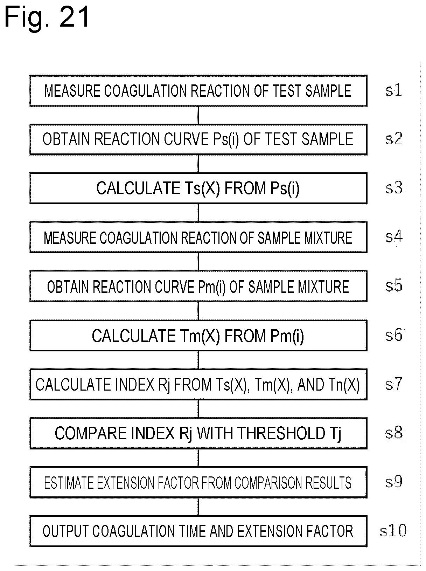

Figure 21 shows an exemplary flow for estimating a coagulation time extension factor for a test sample by the method of the present invention according to one embodiment. Detailed procedures of the flow of Figure 21 will be shown below.

s1: Measuring coagulation reaction of a test sample.
s2: Obtaining reaction curve Ps(i) of the test sample.
s3: Calculating Ts(X) of the test sample from Ps(i) when an extended coagulation time is measured.
s4: Measuring coagulation reaction of a sample mixture.
s5: Obtaining reaction curve Pm(i) of the sample mixture.
s6 Calculating Tm(X) of the sample mixture from Pm(i).
s7: Calculating index Rj from Ts(X), Tm(X), and Tn(X).
(The index Rj is preferably at least one member selected from the group consisting of R1 to R8 mentioned above)
s8: Comparing the index Rj with threshold Tj.
s9: Estimating an extension factor for the test sample from comparison result.
s10: Outputting the coagulation time and the extension factor.

One embodiment of the coagulation time extension factor estimation method of the present invention which abides by the estimation flow described above comprises the steps of:

1-i) obtaining reaction curve Ps(i) of a test sample;
1-ii) obtaining coagulation reaction curve Pm(i) of a sample mixture of the test sample and a normal sample, and coagulation reaction curve Pn(i) of the normal sample, when the test sample has an extended coagulation time;
1-iii) obtaining Ts(X) from Ps(i), obtaining Tm(X) from Pm(i), and obtaining Tn(X) from Pn(i);
2-i) calculating one or more of indexes Rj
wherein $j \geq 1$,
each of the indexes Rj is calculated on the basis of at least one member selected from the group consisting of Ts(X), Tm(X), and Tn(X), and
at least one of the indexes Rj is calculated on the basis of at least Tm(X);
2-ii) comparing the indexes Rj with threshold Tj; and

2-iii) estimating a coagulation time extension factor for the test sample on the basis of the comparison result.

Preferably, the coagulation time extension factor is lupus anticoagulant positivity, coagulation factor deficiency, or coagulation factor inhibitor positivity.

6. Estimation of coagulation time extension factor using machine learning model

In another embodiment of the method of the present invention, a coagulation time extension factor for a test sample can be estimated in accordance with a coagulation time extension factor estimation model constructed by machine learning (machine learning model). Data on Tm(X) from a training sample population and data on the types of coagulation time extension factors (sample species) are used as training data for machine learning. For example, a machine learning model for estimating a coagulation time extension factor for a test sample is constructed by machine learning using a feature which indicates a property or a characteristic of Tm(X) for each sample of a training sample population, for example, the indexes R mentioned above, as an explanatory variable and data on a sample species of each sample of the training sample population as an objective variable.

A blood sample population having a known sample species can be used as the training sample population. In a preferred embodiment, the training sample population includes LA, Def, and Inh as sample species which exhibit an extended coagulation time. Preferably, the sample species Def may be divided into two subspecies, low active Def-1 and anything else Def-2, depending on relatively high or low factor activity. Also preferably, the sample species Inh may be divided into three subspecies, Inh-H having a high titer, Inh-L having a low titer, and Inh-M having a middle titer, or into two subspecies, Inh-H having a high titer and Inh-LM having a low or middle titer, depending on a relatively high or low inhibitor titer. Examples of the feature for use in the explanatory variable include $Ts(X) - Tn(X)$, $Tm(X) - Tn(X)$, the index R mentioned above, for example, R1 to R8 mentioned above (preferably R1(Xa), R1(Xa'), R2(Xb,Xc), and R3(Xd,Xe) mentioned above). Any one of these indexes may be used, or any two or more or any three or more thereof may be used in combination. Further, the index R and a conventionally known index such as PC or RI mentioned above may be used in combination as the feature.

Examples of the machine learning algorithm for use in the construction of the machine learning model include known machine learning algorithms such as support vector machine (SVM), neural network (NN), decision tree, random forest, and k-nearest neighbor algorithm. Data for validation is input to the constructed model to calculate results of estimating a coagulation time extension factor. A model which produces the estimation results most conformable to actual results, for example, a model having the largest accuracy of the estimation results against actual results, or a model having the smallest error between the estimation results and actual results, can be selected as an optimum model.

The constructed machine learning model outputs the results of estimating a coagulation time extension factor (LA, Def, or Inh) for the test sample from the feature (i.e., data corresponding to the explanatory variable mentioned above) which indicates a property or a characteristic of Tm(X) for the test sample.

7. Application to other coagulation reaction measurement methods

The coagulation time extension factor estimation method of the present invention is described above by taking the coagulation reaction measurement based on the amount of scattered light as an example. However, those skilled in the art are capable of applying the method based on the amount of scattered light described above to methods using other coagulation reaction measurement methods (e.g., blood coagulation reaction measurement methods based on a degree of transmission, an absorbance, or a viscosity). Accordingly, such application is included in the scope of the present invention.

8. Program and apparatus

The coagulation time extension factor estimation method of the present invention mentioned above may be automatically performed using a computer program. Thus, one aspect of the present invention provides a program for performing the coagulation time extension factor estimation method of the present invention mentioned above. In a preferred embodiment, the program of the present invention is a program for carrying out the flow of Figure 21 mentioned above. A series of steps in the method of the present invention mentioned above may be automatically performed with an automatic analysis apparatus. Thus, one aspect of the present invention provides an apparatus for performing the coagulation time extension factor estimation method of the present invention mentioned above.

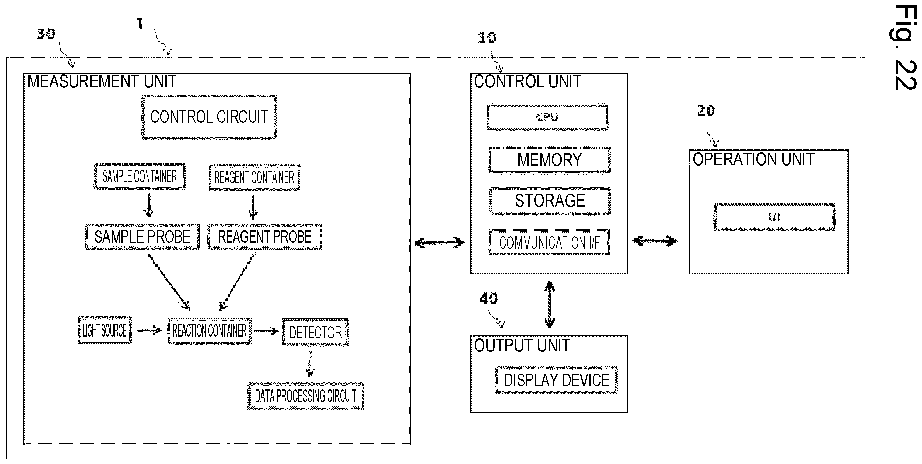

One embodiment of the apparatus of the present invention will be described. One embodiment of the apparatus of the present invention is automatic analysis apparatus 1 as shown in Figure 22. The automatic analysis apparatus 1 has control unit 10, operation unit 20, measurement unit 30, and output unit 40.

The control unit 10 controls the whole performance of the automatic analysis apparatus 1. The control unit 10 may be constituted by, for example, a calculator (e.g., a personal computer). The control unit 10 has CPU, a memory, a storage, a communication interface (I/F), and the like and performs, for example, processing of commands from the operation unit 20, control of the performance of the measurement unit 30, preservation and data analysis of measurement data sent from the measurement unit 30, preservation of analysis results, and control of the output of measurement data or analysis results from the output unit 40. Further, the control unit 10 may be connected with another equipment such as external media or a host computer. In the control unit 10, a calculator which controls the performance of the measurement unit 30 and a calculator which analyzes measurement data may be the same or different.

The operation unit 20 obtains input by an operator and transmits the obtained input information to the control unit 10. The operation unit 20 has, for example, a user interface (UI) such as a keyboard or a touch panel. The output unit 40 outputs analysis results such as measurement data from the measurement unit 30 or coagulation reaction curve P, coagulation reaction end point E, coagulation time, T(X), index R, or results of estimating a coagulation time extension factor for a sample (e.g., LA, Def, Inh, or others) based thereon, under the control of the control unit 10. The output unit 40 has, for example, a display device such as a display.

The measurement unit 30 executes a series of operations for a blood coagulation test and obtains coagulation reaction measurement data on a test sample including a blood sample. The measurement unit 30 has various instruments or analysis modules necessary for a blood coagulation test, for example, a sample container which retains a blood sample, a reagent container which retains a reagent for a test, a reaction container for the reaction of the sample with the reagent, a probe for dispensing the sample, a diluent, the reagent, and the like to the reaction container, a light source, a detector for detecting scattered light or transmitted light from the test sample in the reaction container, a data processing circuit which sends data from the detector to the control unit 10, and a control circuit which controls the operation of the measurement unit 30 in response to the command of the control unit 10.

The control unit 10 estimates a coagulation time extension factor for a sample on the basis of data measured by the measurement unit 30. This analysis may include obtainment of the coagulation reaction curve P and the coagulation time mentioned above, obtainment of coagulation reaction end point E and T(X), calculation of index R, and the like. Further, the control unit 10 may estimate a coagulation time extension factor for a sample using the calculated index R. Alternatively, coagulation reaction curves P and E may be obtained by the control unit 10 on the basis of measurement data from the measurement unit 30, or may be obtained by another equipment, for example, the measurement unit 30 and sent to the control unit 10. The control unit 10 may store the coagulation time of a sample and various parameters for use in the calculation of E, T(X), or R (e.g., a calculation equation for calculating X or R). Also, the control unit 10 may store a parameter for use in the estimation of a coagulation time extension factor for a sample (e.g., the threshold T mentioned above), a machine learning model, and the like. Alternatively, the control unit 10 may capture the parameters or reference values stored in external equipment or network upon analysis.

The present analysis described above may be carried out by a program for performing the method of the present invention. Thus, the control unit 10 may have a program for performing the coagulation time extension factor estimation method of the present invention.

Analysis results obtained by the control unit 10 are sent to the output unit 40 and output. The output may assume an arbitrary form such as display on the screen, transmission to a host computer, or printing. Output information from the output unit can include data on a coagulation reaction curve, coagulation reaction end point E, a coagulation time, T(X), index R, results of estimating a coagulation time extension factor for a sample, and the like. The type of the output information from the output unit may be controlled by the program of the present invention.

Examples

Hereinafter, the present invention will be described in more detail with reference to Examples. However, the present invention is not limited by these Examples.

Method

1) Sample

The normal sample (NP) used was CRYOcheck Pooled Normal Plasma (N = 1) from Precision BioLogic Incorporated (N = 1 in total).

The LA-positive samples (LA) used were Positive Lupus Anticoagulant Plasma (6 lots, N = 1 for each) from George King Bio-Medical, Inc. and plasma (N = 1) obtained by diluting one of the lots 10-fold with normal plasma (N = 7 in total; L-1 to L-7).

FVIII-deficient plasma samples (Def) used were Factor VIII Deficient (4 lots, N = 1 for each) from George King Bio-Medical, Inc., which was confirmed to have a FVIII activity value of less than 1%, and a total of four plasma samples (N = 1 for each) obtained by adding 1% and 5% normal plasma to each of two of the lots (N = 8 in total; D-1 to D-8).

The FVIII inhibitor-positive samples (Inh) used were Factor VIII Deficient with Inhibitor (3 lots, N = 1 for each) from George King Bio-Medical, Inc. and a total of seven plasma samples (N = 1 for each) obtained by diluting these three lots 2-fold to 24-fold with normal plasma (N = 10 in total; I-1 to I-10). The inhibitor titers (unit: BU/mL; hereinafter, also simply referred to as titers) of the samples were determined in accordance with the product specification. The titers of the samples diluted with normal plasma were calculated on the basis of the titers before dilution and the dilution ratios.

A sample mixture of each sample was prepared using the normal sample (NP).

2) Coagulation reaction measurement

The reagents for measurements used were Coagpia APTT-N APTT reagent (manufactured by Sekisui Medical Co., Ltd.), which was an APTT measurement reagent, as the first reagent and Coagpia APTT-N calcium chloride solution (25 mM calcium chloride solution, manufactured by Sekisui Medical Co., Ltd.) as the second reagent. The coagulation reaction measurement of a test sample including each sample was performed using automatic blood coagulation analysis apparatus CP3000 (manufactured by Sekisui Medical Co., Ltd.). 50 μL of the sample was warmed in a cuvette at 37°C for 45 seconds. Then, 50 μL of the first reagent of approximately 37°C was added thereto, and further, 50 μL of the second reagent was added after a lapse of 171 seconds to start coagulation reaction. The reaction was performed at 37°C. The coagulation reaction was measured by irradiating the cuvette with light at a wavelength of 660 nm from LED as a light source, followed by the photometry of the amount of scattered light of 90-degree side scattered light at 0.1-second intervals. The measurement time was 360 seconds. In the case of warming the sample for measurement, the sample before measurement reagent addition was warmed at 37°C for 12 minutes through the use of the sample warming function of the analysis apparatus.

3) Obtainment of coagulation reaction curve

The photometric data from each sample was subjected to smoothing treatment including noise removal and then treated by zero point adjustment such that the amount of scattered light at the start of photometry was 0 to prepare coagulation reaction curve P(i).

4) Detection of coagulation reaction end point E and determination of APTT

P(i) at the earliest point in time when integrated ratio Z(i) of coagulation reaction curve P(i) (see PCT/JP2020/048676) was less than integrated ratio threshold Zs was detected as coagulation reaction end point **E.** The integrated ratio threshold Zs used was 1.001. The point in time when P(i) reached 50% of E was calculated as T(50) and determined as APTT. The integrated ratio Z(i) at the point in time, **i,** was calculated as follows.

$$\text{Integrated ratio } Z(i) = Pb(i) / Pa(i)$$

$$Pa(i) = \text{Sum of } P(i-20) \text{ to } P(i-1)$$

$$Pb(i) = \text{Sum of } P(i + 1) \text{ to } P(i + 20)$$

5) Calculation of T(X)

Time T(X) when P(i) reached X% of E detected in 3) was calculated for each sample on the basis of the equation given below. 100 Xs were set from 1 to 100 in increments of 1, and T(1) to T(100) were calculated. T(50) at X = 50 corresponds to APTT.

$$P(T(X)) = E \times X\%$$

Coagulation reaction of sample

Figure 3 shows P(i) (ordinate: the amount of scattered light) (upper diagrams) and T(X) (lower diagrams) of each test sample of LA, Def, or Inh. Figure 3A illustrates data on LA, Figure 3B illustrates data on Def, and Figure 3C illustrates data on Inh. Each diagram also shows data on the normal sample (NP) (dotted line).

Figure 4 shows P(i) (ordinate: the amount of scattered light) (upper diagrams) and T(X) (lower diagrams) of a sample mixture (mixing ratio; test sample:NP = 1:1) prepared from each test sample of LA, Def, or Inh. Figure 4A illustrates data on LA, Figure 4B illustrates data on Def, and Figure 4C illustrates data on Inh. Each diagram also shows data on the normal sample (NP) (dotted line).

Table 1 shows the inhibitor titer and APTT of each test sample (LA, Def, and Inh) as well as APTT (APTT before warming, a) of the sample mixture (mixing ratio; test sample:NP = 1:1) prepared from each test sample, APTT (APTT after warming, b) of a sample obtained by the warming treatment (37°C, 12 min) of the sample mixture, the difference in APTT (b - a) of the sample mixture between before and after warming.

[Table 1]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |
| a APTT before warming, mixing ratio of 1:1 [sec] | 48 | 41 | 61 | 57 | 52 | 122 | 84 | 35 | 33 | 35 | 33 | 35 | 34 | 32 | 34 | 32 | 32 | 32 | 36 | 38 | 46 | 49 | 64 | 69 | 45 |
| b APTT after warming, mixing ratio of 1:1 [sec] | 47 | 41 | 60 | 56 | 51 | 117 | 83 | 35 | 34 | 35 | 35 | 35 | 34 | 32 | 33 | 32 | 34 | 35 | 50 | 54 | 79 | 85 | 109 | 114 | 73 |
| b-a: difference in APTT [sec] | -1 | -1 | -1 | 0 | -1 | -5 | -2 | 0 | 0 | 1 | 2 | 1 | 0 | 0 | 0 | 0 | 2 | 2 | 15 | 16 | 33 | 36 | 44 | 45 | 29 |

Figure 5A shows the APTT of each test sample. LA, Def, and Inh had overlapping APTT distribution ranges, confirming that the sample species cannot be discriminated from each other using APTT.

As for the difference in APTT (b - a) of each sample mixture between before and after warming, Figure 5B shows the distribution of each sample species, and Figure 5C shows a plot of the difference against the titer of the Inh sample. For Inh, the warming treatment of the sample mixture resulted in the difference in APTT (b - a) in some samples. By contrast, for LA and Def, APTT was rarely changed by the warming treatment of the sample mixture. The difference in APTT (b - a) of Inh was increased depending on the titer and however, was not in a linear relationship. Inh having a titer of 5 or less was not able to be discriminated from Def using the difference in APTT (b - a).

Example 1

Figure 6A shows P(i) of three test samples differing in sample species but having the same level of APTT (around 100 seconds), together with data on NP. The shape of P(i) differed among the sample species, even though APTT was at the same level. Def and Inh tended to have a similar shape of P(i), and their curves rose relatively gently. Although LA had extended APTT, its curve rose relatively sharply.

On the other hand, the coagulation reaction of each sample mixture exhibited different behavior from that of the test sample. Figure 6B shows P(i) of the sample mixture (mixing ratio; test sample:NP = 1:1) prepared from each of the three test samples shown in Figure 6A. For the sample mixtures derived from Def and Inh (Def-M11 and Inh-M11), the shape of P(i) was closer to that of NP so that APTT fell within a normal range (from 24 to 39 seconds). On the other hand, the sample mixture derived from LA (LA-M11) was similar in the shape of P(i) to the original sample (LA in Figure 6A) and still had extended APTT, albeit reduction in APTT was seen.

Figures 7A to 7C illustrate T(X) (Ts(X) and Tm(X)) of the test samples and the sample mixtures shown in Figures 6A and 6B. Figure 7A shows data on LA and its sample mixture LA-M11, Figure 7B shows data on Def and its sample mixture Def-M11, and Figure 7C shows data on Inh and its sample mixture Inh-M11. Each diagram also shows Tn(X) for NP. Relative placement of curves which indicate Ts(X), Tm(X), and Tn(X) differed among sample species, mainly due to downward (direction closer to Tn(X)) shift of Tm(X) compared with Ts(X). The downward shift of Tm(X) was small for LA (Figure 7A) and large for Def and Inh (Figures 7B and 7C). These results suggest that the difference between Ts(X) and Tm(X) [Ts(X) - Tm(X)] and the difference between Tm(X) and Tn(X) [Tm(X) - Tn(X)] at arbitrary X differ depending on a sample species. In short, the value of [Ts(X) - Tm(X)] of Def and Inh is larger than that of LA, whereas the value of [Tm(X) - Tn(X)] of Def and Inh is smaller than that of LA. Also, there is a small difference in [Ts(X) - Tm(X)] or [Tm(X) - Tn(X)] between Def and Inh.

Example 2

Sample mixtures with a mixing ratio (test sample:NP) of 1:9, 1:1, or 9:1 were prepared for the test samples (LA, Def, and Inh) shown in Figure 6A. Tm(X) of each sample mixture was calculated. Ts(X) and Tn(X) used were the same values as in Example 1. The following index was calculated for each sample mixture using Tm(X), Ts(X), and Tn(X) .

$$FT1(X) = (Tm(X) - Tn(X)) / (Ts(X) - Tn(X)) \times 100$$

Figures 8A to 8C each illustrate a plot of FT1(X) calculated for the sample mixture derived from each test sample shown in Figure 6A. Figure 8A shows data on the sample mixture with a mixing ratio (test sample:NP) of 1:9 (1:9 sample mixture), Figure 8B shows data on the sample mixture with a mixing ratio (test sample:NP) of 1:1 (1:1 sample mixture), and Figure 8C shows data on the sample mixture with a mixing ratio (test sample:NP) of 9:1 (9:1 sample mixture). In each diagram, the dash-dotted line depicts the LA-derived sample, the dash-double dot line depicts the Def-derived sample, and the solid line depicts the Inh-derived sample. In Figures 8A to 8C, FT1(X) for LA was larger than that for Def and Inh and was nearly in a horizontal state with small change in the range of X from 0 to around 60. On the other hand, FT1(X) for both Def and Inh decreased with increase in X and was smallest around X = 95. FT1(X) for Inh was slightly larger than that for Def, and the difference in FT1(X) therebetween tended to widen with decrease in X. These results suggested that: LA can be discriminated from Def and Inh on the basis of FT1(X); and there is a possibility that Def and Inh can be discriminated from each other.

As seen from Figure 8, FT1(X) for Def and Inh was smallest around X = 95. Therefore, the following index was calculated for each sample mixture.

$$\Delta FT1_{X-95} = [FT1(X) - FT1(95)]$$

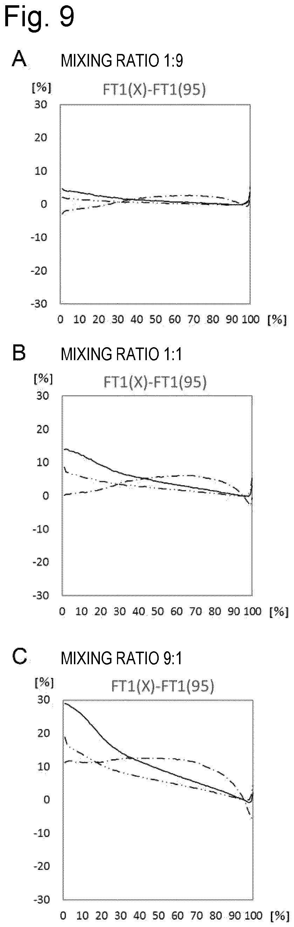

Figures 9A to 9C show plots of $\Delta FT1_{X-95}$ for the sample mixtures of Figures 8A to 8C, respectively. Particularly, when X was 40 or less, the value of $\Delta FT1_{X-95}$ differed between Def and Inh. Further, a higher proportion of the test sample in the sample mixture increased the difference in $\Delta FT1_{X-95}$ between Def and Inh. These results suggested that Def and Inh can be discriminated from each other on the basis of $\Delta FT1_{X-95}$.

Example 3

Sample mixtures with a mixing ratio (test sample:NP) of 1:1 were prepared for a total of three samples involving one sample of Def (APTT: 108 seconds) and two samples of Inh differing in titer (a titer of 18 and a titer of 5; APTT: 105 seconds and 57 seconds, respectively). Tm(X) of each sample mixture was calculated. Tn(X) used was the same value as in Example 1. The following index was calculated for each sample mixture using Tm(X) and Tn(X) .

$$FT2(X) = Tm(X) / Tn(X) \times 100$$

FT3(X,95) = (Tm(X) - Tm(95)) / (Tn(X) - Tn(95)) $\times$ 100

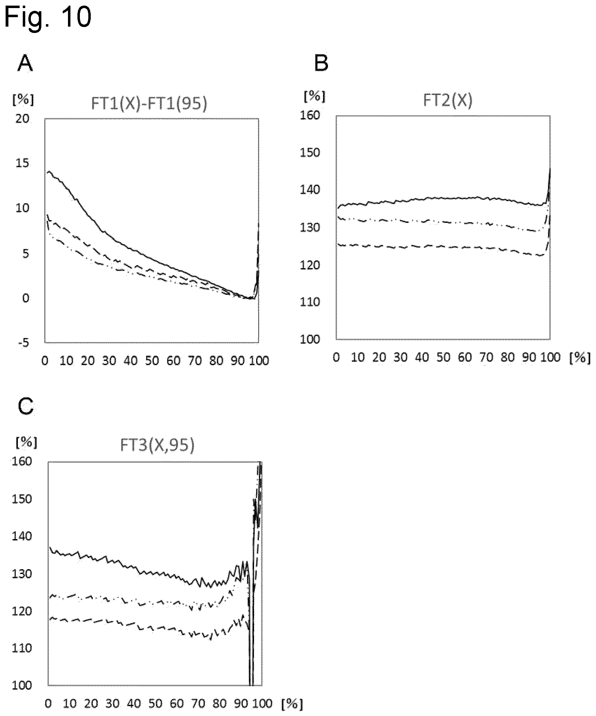

Figures 10A to 10C show plots of $\Delta FT1_{X-95}$ (Figure 10A), FT2(X) (Figure 10B), and FT3(X,95) (Figure 10C) for the three samples. In each diagram, the dash-double dot line depicts the Def-derived sample mixture, and the solid line and the broken line depict the Inh-derived sample mixtures with inhibitor titers of 18 and 5, respectively.

$\Delta FT1_{X-95}$ for Inh having a titer of 5 fell between Def and Inh having a titer of 18 and however, had the difficulty in discriminating this Inh from Def. The value of FT2(X) differed between Def and Inh, whereas the values for Def and Inh having a titer of 18 were closer to each other with decrease in X. The value of FT3(X,95) differed between Def and Inh, and furthermore, the values for Def and Inh having a titer of 18 were larger with decrease in X (particularly, within the X range of approximately 40 or less). These results suggested that FT3(X) is suitable as an index for discriminating Def and Inh from each other in more detail.

Example 4

The discrimination of LA, Def, and Inh was attempted using the indexes FT1(X), $\Delta FT1_{X-95}$, and FT3(X,95) mentioned in Examples 2 and 3. Tm(X) was calculated using sample mixtures with a mixing ratio (test sample:NP) of 1:1.

1) FT1(X) and $\Delta FT1$

The following indexes 1 to 3 were calculated for the test samples (LA, Def, and Inh).

Index 1; FT1(95)
Index 2; FT1(10)
Index 3;

$$\Delta FT1_{10-95} = [FT1(10) - FT1(95)]$$

$$[FT1(X) = (Tm(X) - Tn(X)) / (Ts(X) - Tn(X)) \times 100]$$

A mean and standard deviation (SD) of Def(D-1 to D-8) was determined for each of the indexes 1 to 3, and the mean + 4SD was set to a threshold for each index.

Figures 11A and 11B show the calculated indexes 1 and 2 of each test sample, Figure 11C shows the index 3 of Def and Inh, and Figure 11D shows a plot of the index 3 of Inh against a titer. In each diagram, the dotted line depicts the threshold.

The value of the index 1 was higher in LA than in Def or Inh (Figure 11A). The index 2 and the index 3 of Inh tended to be higher than those of Def and however, were not able to discriminate some Inh samples from Def (Figures 11B and 11C). The index 3 of Inh having a titer of 5 or less was lower than the threshold and was not able to discriminate this Inh from Def, whereas the index 3 for Inh having a titer of higher than 5 increased along with the titer and exceeded the threshold, showing a distribution different from that of Def (Figure 11D).

The indexes 1 to 3 of each test sample were compared with the threshold, and a sample species of the test sample was estimated according to the following criteria on the basis of the comparison results.

<Estimation criteria>

(i) Index 1 > threshold: "LA"
The remaining samples were further divided in the following order.
(ii) Index 2 < threshold and index 3 < threshold: "Def"
(iii) Index 2 < threshold and index 3 $\geq$ threshold: "Inh (In2)"
(iv) Index 2 $\geq$ threshold and index 3 $\geq$ threshold: "Inh (In1)"

Table 2 shows the indexes 1 to 3 of each test sample, results of comparing these indexes with the threshold, and the estimated sample species. The present estimation criteria enabled LA to be discriminated from the other sample species (Def and Inh) and enabled Inh having a titer exceeding 5 (In2; I-4 to I-7 and I-10) to be accurately identified. The index 2 enabled high-titer Inh having a titer of 92 or more (In1; I-8 to I-9) to be identified. On the other hand, low-titer Inh having a titer of 5 or less (I-1 to I-3) was discriminated from Def.

[Table 2]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | FT1(95) | 38 | 94 | 71 | 86 | 70 | 56 | 116 | 73 | 23 | 19 | 15 | 11 | 10 | 8 | 5 | 5 | 33 | 22 | 18 | 8 | 10 | 15 | 18 | 28 | 32 | 10 |
| 2 | FT1(10) | 46 | 109 | 68 | 138 | 107 | 67 | 147 | 74 | 30 | 25 | 22 | 17 | 18 | 13 | 11 | 11 | 40 | 29 | 25 | 20 | 25 | 35 | 39 | 50 | 53 | 22 |
| 3 | FT1(10)-FT1(95) | 10 | 15 | -4 | 52 | 38 | 10 | 31 | 1 | 7 | 6 | 7 | 6 | 8 | 6 | 6 | 6 | 7 | 7 | 8 | 12 | 15 | 19 | 21 | 22 | 21 | 11 |
| Index1 | Index > Threshold: given + | | + | + | + | + | + | + | + | - | - | - | - | - | - | - | - | - | - | - | - | - | - | - | - | - | - |
| Index2 | Index < Threshold: given + | | - | - | - | - | - | - | - | + | + | + | + | + | + | + | + | + | + | + | + | + | + | + | - | - | + |
| Index3 | Index < Threshold: given + | | - | + | - | - | - | - | + | + | + | + | + | + | + | + | + | + | + | + | - | - | - | - | - | - | - |
| | Determination | | LA | LA | LA | LA | LA | LA | LA | Def | Def | Def | Def | Def | Def | Def | Def | Def | Def | Def | In2 | In2 | In2 | In2 | In1 | In1 | In2 |

2) FT1(X), ΔFT1, and FT3(X$_1$,X$_2$)

The following indexes 1 to 3 were calculated for the test samples (LA, Def, and Inh).

Index 1; FT1(95)
Index 2;

$$\Delta FT1_{10-95} = [FT1(10) - FT1(95)]$$

Index 3; FT3(10,95)

$$[FT1(X) = (Tm(X) - Tn(X)) / (Ts(X) - Tn(X)) \times 100$$

$$FT3(X_1, X_2) = (Tm(X_1) - Tm(X_2)) / (Tn(X_1) - Tn(X_2)) \times 100]$$

A mean + 4SD of Def(D-1 to D-8) was set to a threshold for each of the indexes 1 and 2. A mean - 2SD of Def(D-1 to D-8) was set to a threshold for the index 3.

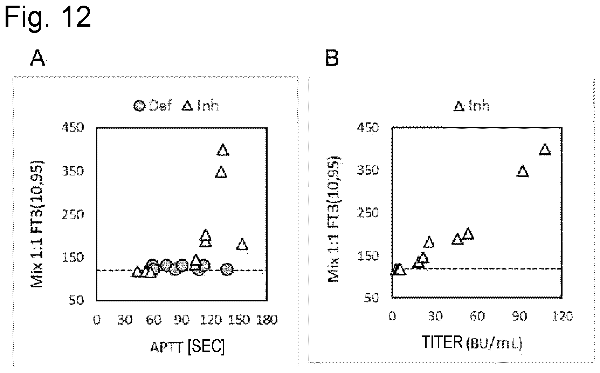

Figures 12A shows the index 3 of Def and Inh, and Figure 12B shows a plot of the index 3 of Inh against a titer. In each diagram, the dotted line depicts the threshold. The index 3 of Def rarely varied among the samples. The Inh samples were divided into a sample with index 3 lower than that of Def, a sample with index 3 higher than that of Def, and a sample with the same level of index 3 as that of Def (Figure 12A). The index 3 of Inh was proportional to a titer and was smaller than the threshold in three samples having a titer of 5 or less (Figure 12B).

The indexes 1 to 3 of each test sample were compared with the threshold, and a sample species of the test sample was estimated according to the following criteria on the basis of the comparison results.

<Estimation criteria>

 (i) Index 1 > threshold: "LA"
 The remaining samples were further divided in the following order.
 (ii) Index 2 < threshold and index 3 $\geq$ threshold: "Def"
 (iii) Index 2 $\geq$ threshold and index 3 $\geq$ threshold: "Inh (In1)"
 (iv) Index 2 < threshold and index 3 < threshold: "Inh (In2)"

Table 3 shows the indexes 1 to 3 of each test sample, results of comparing these indexes with the threshold, and the estimated sample species. The present estimation criteria enabled LA, Def, and Inh to be discriminated from each other and enabled low-titer Inh having a titer of 5 or less (In2; I-1 to I-3) to be discriminated from the other Inh samples (In1; I-4 to I-10).

[Table 3]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | FT1(95) | 38 | 94 | 71 | 86 | 70 | 56 | 116 | 73 | 23 | 19 | 15 | 11 | 10 | 8 | 5 | 5 | 33 | 22 | 18 | 8 | 10 | 15 | 18 | 28 | 32 | 10 |
| 2 | FT1(10)-FT1(95) | 10 | 15 | -4 | 52 | 38 | 10 | 31 | 1 | 7 | 6 | 7 | 6 | 8 | 6 | 6 | 6 | 7 | 7 | 8 | 12 | 15 | 19 | 21 | 22 | 21 | 11 |
| 3 | FT3(10,95) | 120 | 229 | 213 | 323 | 296 | 231 | 639 | 439 | 133 | 125 | 132 | 125 | 133 | 124 | 134 | 125 | 118 | 118 | 117 | 136 | 146 | 189 | 203 | 349 | 400 | 182 |
| Index1 | Index > Threshold: given + | | + | + | + | + | + | + | + | - | - | - | - | - | - | - | - | - | - | - | - | - | - | - | - | - | - |
| Index2 | Index < Threshold: given + | | - | + | - | - | - | - | + | + | + | + | + | + | + | + | + | - | - | - | - | - | - | - | - | - | - |
| Index3 | Index < Threshold: given + | | - | - | - | - | - | - | - | - | - | - | - | - | - | - | - | + | + | + | - | - | - | - | - | - | - |
| | Determination | | LA | LA | LA | LA | LA | LA | LA | Def | Def | Def | Def | Def | Def | Def | Def | In2 | In2 | In2 | In1 | In1 | In1 | In1 | In1 | In1 | In1 |

3) FT1(X), $\Delta$FT1, and FT3($X_1$,$X_2$)

The indexes 1 to 3 calculated in 2) described above were used. The same threshold as in 2) described above was used for the indexes 1 and 2, and two thresholds, threshold L: a mean - 2SD of Def(D-1 to D-8) and threshold H: a mean + 4SD of Def(D-1 to D-8), were used for the index 3. The indexes 1 to 3 were compared with the threshold, and a sample species of the test sample was estimated according to the following criteria on the basis of the comparison results.

<Estimation criteria>

 (i) Index 1 > threshold: "LA"
 The remaining samples were further divided in the following order.
 (ii) Index 2 < threshold and index 3 $\geq$ threshold L: "Def"
 (iii) Index 3 > threshold H: "Inh (In1)"
 (iv) Index 3 $\geq$ threshold L and index 3 $\leq$ threshold H: "Inh (In2)"
 (v) Index 3 < threshold L: "Inh (In3)"

Table 4 shows the indexes 1 to 3 of each test sample, results of comparing these indexes with the threshold, and the estimated sample species. The present estimation criteria enabled LA, Def, and Inh to be discriminated from each other and enabled Inh to be classified into three stages, a low titer ($\leq$ 5) (In3), a middle titer (In2), and a high titer ($\geq$ 26) (In1), depending on a titer.

[Table 4]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | FT1(95) | 38 | 94 | 71 | 86 | 70 | 56 | 116 | 73 | 23 | 19 | 15 | 11 | 10 | 8 | 5 | 5 | 33 | 22 | 18 | 8 | 10 | 15 | 18 | 28 | 32 | 10 |
| 2 | FT1(10)-FT1(95) | 10 | 15 | -4 | 52 | 38 | 10 | 31 | 1 | 7 | 6 | 7 | 6 | 8 | 6 | 6 | 6 | 7 | 7 | 8 | 12 | 15 | 19 | 21 | 22 | 21 | 11 |
| 3 | FT3(10,95) | 120 | 229 | 213 | 323 | 296 | 231 | 639 | 439 | 133 | 125 | 132 | 125 | 133 | 124 | 134 | 125 | 118 | 118 | 117 | 136 | 146 | 189 | 203 | 349 | 400 | 182 |
| 3 | FT3(10,95) | 147 | 229 | 213 | 323 | 296 | 231 | 639 | 439 | 133 | 125 | 132 | 125 | 133 | 124 | 134 | 125 | 118 | 118 | 117 | 136 | 146 | 189 | 203 | 349 | 400 | 182 |
| Index1 | Index > Threshold: given + | | + | + | + | + | + | + | + | - | - | - | - | - | - | - | - | - | - | - | - | - | - | - | - | - | - |
| Index2 | Index < Threshold: given + | | - | + | - | - | - | - | + | + | + | + | + | + | + | + | + | + | + | + | - | - | - | - | - | - | - |
| Index3 | Index < Threshold L: given + | | - | - | - | - | - | - | - | - | - | - | - | - | - | - | - | + | + | + | - | - | - | - | - | - | - |
| Index3 | Index > Threshold H: given + | | + | + | + | + | + | + | + | - | - | - | - | - | - | - | - | - | - | - | - | - | + | + | + | + | + |
| | Determination | | LA | LA | LA | LA | LA | LA | LA | Def | Def | Def | Def | Def | Def | Def | Def | In3 | In3 | In3 | In2 | In2 | In1 | In1 | In1 | In1 | In1 |

4) 9:1 Sample mixture

Indexes 1 and 2 given below were calculated for the test samples (LA, Def, and Inh). Tm(X) was calculated using sample mixtures with a mixing ratio (test sample:NP) of 9:1.

Index 1; FT1(10)
Index 2; FT3(10,85)

$$[FT1(X) = (Tm(X) - Tn(X)) / (Ts(X) - Tn(X)) \times 100$$

$$FT3(X_1, X_2) = (Tm(X_1) - Tm(X_2)) / (Tn(X_1) - Tn(X_2)) \times 100]$$

A mean + 4SD of Def(D-1 to D-8) was set to a threshold for the index 1. Two thresholds, threshold L: a mean - 2.5SD of Def(D-1 to D-8) and threshold H: a mean + 2.5SD of Def(D-1 to D-8), were set for the index 2.

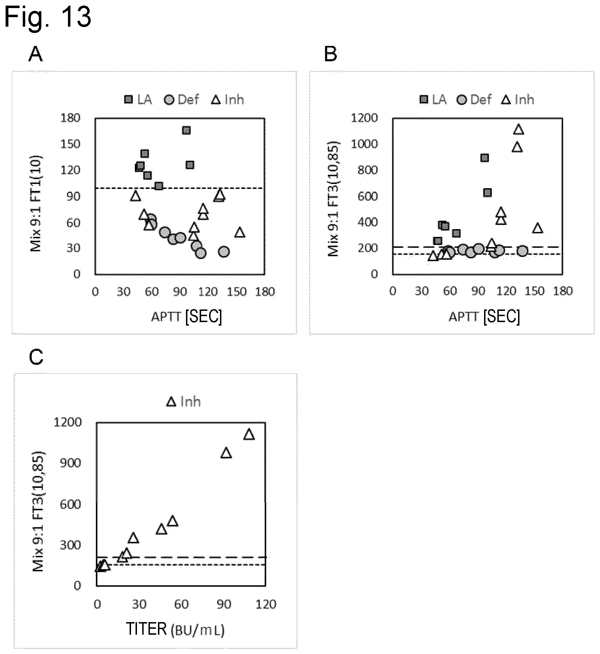

Figure 13A and 13B show the calculated indexes 1 and 2 of each test sample, and Figure 13C shows a plot of the index 2 of Inh against a titer. In Figure 13A, the dotted line depicts the threshold of the index 1. In Figures 13B and 13C, the dotted line depicts the threshold L of the index 2, and the broken line depicts the threshold H of the index 2. The value of the index 1 was higher in LA than in Def or Inh (Figure 13A). The index 2 of LA and Inh tended to be higher than that of Def, though the index 2 was not able to discriminate some Inh samples from Def (Figure 13B). The index 2 of Inh increased in proportion to a titer and however, in the case of a low titer, did not exceed the threshold L or the threshold H and was not able to discriminate this Inf from Def (Figure 13C).

The indexes 1 and 2 of each test sample were compared with the threshold, and a sample species of the test sample was estimated according to the following criteria on the basis of the comparison results.

<Estimation criteria>

(i) Index 1 > threshold: "LA"
The remaining samples were further divided in the following order.
(ii) Index 2 $\geq$ threshold L and index 2 $\leq$ threshold H: "Def"
(iii) Index 2 > threshold H: "Inh (In1)"
(iv) Index 2 < threshold L: "Inh (In2)"

Table 5 shows the indexes 1 and 2 of each test sample, results of comparing these indexes with the threshold, and the estimated sample species. The present estimation criteria enabled LA, Def, and Inh to be discriminated from each other and enabled low-titer ($\leq$ 5) Inh (In2; I-1 to I-3) to be identified.

There is a possibility that the present estimation criteria cannot discriminate Inh with index 2 equal to or more than the threshold L and equal to or less than the threshold H, if present, from Def. However, this approach has an advantage over a conventional method because the approach is capable of identifying LA or some Inh samples without requiring a sample incubated for a long time, which is used for delayed reaction in a cross mixing test.

[Table 5]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | FT1(10) | 99 | 123 | 125 | 139 | 113 | 101 | 166 | 126 | 64 | 59 | 50 | 41 | 43 | 33 | 25 | 26 | 91 | 70 | 58 | 45 | 55 | 69 | 76 | 90 | 92 | 49 |
| 2 | FT3(10,85) | 155 | 259 | 259 | 382 | 374 | 317 | 895 | 626 | 183 | 172 | 194 | 170 | 198 | 170 | 188 | 182 | 145 | 154 | 154 | 216 | 244 | 424 | 480 | 982 | 1117 | 359 |
| 2 | FT3(10,85) | 209 | 259 | 259 | 382 | 374 | 317 | 895 | 626 | 183 | 172 | 194 | 170 | 198 | 170 | 188 | 182 | 145 | 154 | 154 | 216 | 244 | 424 | 480 | 982 | 1117 | 359 |
| Index1 | Index > Threshold: given + | | + | + | + | + | + | + | + | - | - | - | - | - | - | - | - | - | - | - | - | - | - | - | - | - | - |
| Index2 | Index < Threshold L: given + | | - | - | - | - | - | - | - | - | - | - | - | - | - | - | - | + | + | + | - | - | - | - | - | - | - |
| Index2 | Index > Threshold H: given + | | + | + | + | + | + | + | + | - | - | - | - | - | - | - | - | - | - | - | + | + | + | + | + | + | + |
| | Determination | | LA | LA | LA | LA | LA | LA | LA | Def | Def | Def | Def | Def | Def | Def | Def | In2 | In2 | In2 | In1 | In1 | In1 | In1 | In1 | In1 | In1 |

5) 1:9 sample mixture

Indexes 1 to 4 given below were calculated for the test samples (LA, Def, and Inh). Tm(X) was calculated using sample mixtures with a mixing ratio (test sample:NP) of 1:9.

Index 1; FT1(95)
Index 2; FT1(10)
Index 3;

$$\Delta FT1_{10-95} = [FT1(10) - FT1(95)]$$

Index 4; FT3(10,95)

$$[FT1(X) = (Tm(X) - Tn(X)) / (Ts(X) - Tn(X)) \times 100$$

$$FT3(X_1, X_2) = (Tm(X_1) - Tm(X_2)) / (Tn(X_1) - Tn(X_2)) \times 100]$$

A mean + 3SD of Def(D-1 to D-8) was set to a threshold for the indexes 1 and 4. A mean + 2SD of Def(D-1 to D-8) was set to a threshold for the indexes 2 and 3.

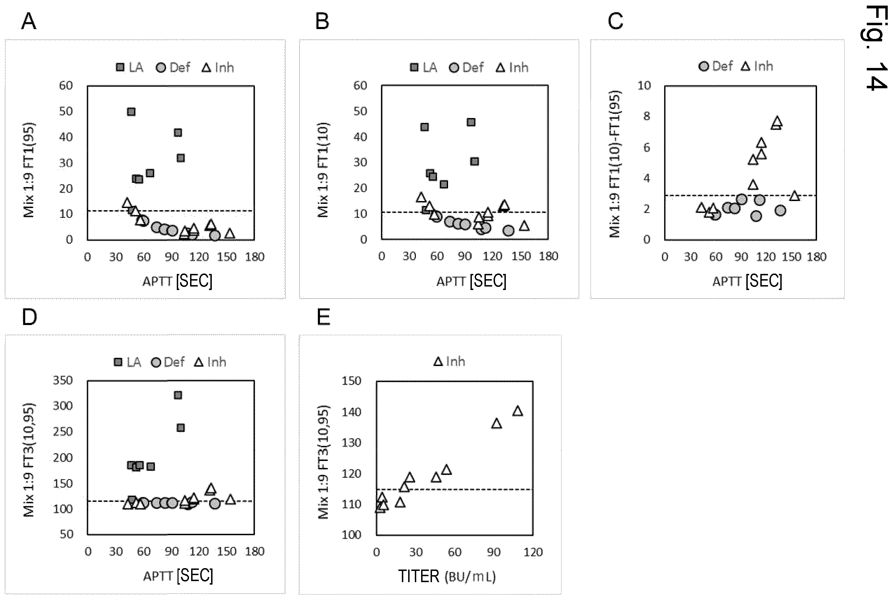

Figures 14A to 14D show the calculated indexes 1 to 4 of each test sample or Def and Inh, and Figure 14E shows a plot of the index 4 of Inh against a titer. In each diagram, the dotted line depicts the threshold.

The indexes 1, 2, and 4 tended to be higher in LA than in Def or Inh and however, were not able to discriminate Def and Inh from each other (Figures 14A, 14B, and 14D). The index 3 of Inh tended to be higher than that of Def, though the index 3 was not able to discriminate some Inh samples from Def (Figure 14C). The index 4 of Inh tended to increase in proportion to a titer (Figure 14E).

The indexes 1 to 4 of each test sample were compared with the threshold, and a sample species of the test sample was estimated according to the following criteria on the basis of the comparison results.

<Estimation criteria>

(i) Index 1 > threshold and index 4 > threshold: "LA"
The remaining samples were further divided in the following order.
(ii) Index 2 < threshold and index 3 < threshold: "Def"
(iii) Index 3 < threshold and index 4 ≤ threshold: "Inh (In3)"
(iv) Index 3 ≥ threshold and index 4 ≤ threshold: "Inh (In2)"
(v) Index 3 ≥ threshold and index 4 > threshold: "Inh (In1)"

Table 6 shows the indexes 1 to 4 of each test sample, results of comparing these indexes with the threshold, and the estimated sample species. The present estimation criteria enabled LA, Def, and Inh to be accurately identified except for one Inh sample (I-3). Further, the present criteria permit estimation of sample species using small amounts of test samples because a sample mixture with a low proportion of a test sample can be used. This may bring about the advantage of being capable of reducing the amount of blood collected from a test subject. For example, when a test sample has an extended coagulation time, it is possible to estimate a sample species using the remaining sample without the need of newly collecting blood from the test subject.

[Table 6]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | FT1(95) | 11 | 50 | 12 | 24 | 23 | 26 | 42 | 32 | 8 | 7 | 5 | 4 | 4 | 3 | 2 | 2 | 15 | 11 | 8 | 2 | 4 | 4 | 4 | 6 | 6 | 3 |
| 2 | FT1(10) | 11 | 44 | 12 | 26 | 25 | 22 | 46 | 31 | 9 | 9 | 7 | 6 | 6 | 4 | 5 | 4 | 17 | 13 | 10 | 6 | 9 | 9 | 11 | 13 | 14 | 5 |
| 3 | FT1(10)-FT1 (95) | 3 | -6 | 0 | 2 | 1 | -5 | 4 | -1 | 2 | 2 | 2 | 2 | 3 | 2 | 3 | 2 | 2 | 2 | 2 | 4 | 5 | 6 | 6 | 8 | 8 | 3 |
| 4 | FT3(10,95) | 115 | 186 | 117 | 181 | 186 | 183 | 321 | 258 | 112 | 112 | 112 | 112 | 112 | 110 | 113 | 111 | 109 | 112 | 110 | 111 | 116 | 119 | 121 | 136 | 140 | 119 |
| Index1 | Index > Threshold: given + | | + | + | + | + | + | + | + | - | - | - | - | - | - | - | - | + | - | - | - | - | - | - | - | - | - |
| Index2 | Index < Threshold: given + | | - | - | - | - | - | - | - | + | + | + | + | + | + | + | + | - | - | + | + | + | + | + | - | - | + |
| Index3 | Index < Threshold: given + | | + | + | + | + | + | - | + | + | + | + | + | + | + | + | + | + | + | + | - | - | - | - | - | - | - |
| Index4 | Index > Threshold: given + | | + | + | + | + | + | + | + | - | - | - | - | - | - | - | - | - | - | - | - | + | + | + | + | + | + |
| | Determination | | LA | LA | LA | LA | LA | LA | LA | Def | Def | Def | Def | Def | Def | Def | Def | In3 | In3 | Def | In2 | In1 | In1 | In1 | In1 | In1 | In1 |

Example 5

1) 1:1 sample mixture

Indexes 1 to 3 given below were calculated for the test samples (LA, Def, and Inh). Tm(X) was calculated using sample mixtures with a mixing ratio (test sample:NP) of 1:1.

Index 1 = FT4s(5,35,50)
Index 2 = FT4m(5,35,50)
Index 3 = FT5(5,35)

$$[FT4s(X_1, X_2, X_3) = [(Ts(X_2) - Ts(X_1)) / Ts(X_3)] \times 100$$

$$FT4m(X_1, X_2, X_3) = [(Tm(X_2) - Tm(X_1)] / Tm(X_3)] \times 100$$

$$FT5(X_1, X_2) = [(Tm(X_2) - Tm(X_1)) \times 100]$$

A mean and standard deviation (SD) of Def(D-1 to D-8) was determined for each of the indexes 1 to 3. The mean + 2SD was set to a threshold for the index 1, the mean - 2SD was set to a threshold for the index 2, and two thresholds, threshold L: the mean - 1.5SD and threshold H: the mean + 2SD, were used for the index 3. Figures 15A, 15B, and 15C show the calculated indexes 1 to 3 of each test sample, and Figure 15D shows a plot of the index 3 of Inh against a titer. In each diagram, the dotted line and the broken line depict the threshold.

The indexes 1 to 3 were compared with the threshold, and a sample species of the test sample was estimated according to the following criteria on the basis of the comparison results.

<Estimation criteria>

(i) Index 1 < threshold and index 2 > threshold: "LA"
The remaining samples were further divided in the following order.
(ii) Index 3 $\geq$ threshold L and index 3 < threshold H: "Def"
(iii) Index 3 < threshold L: "Inh (In3)"
(iv) Index 2 $\leq$ threshold and index 3 $\geq$ threshold H: "Inh (In2)"
(v) Index 2 > threshold and index 3 $\geq$ threshold H: "Inh (In1)"

Table 7 shows the indexes 1 to 3 of each test sample, results of comparing these indexes with the threshold, and the estimated sample species. The present estimation criteria enabled LA, Def, and Inh to be discriminated from each other and enabled Inh to be identified depending on a titer.

[Table 7]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | FT4s(5,35,50) | 22 | 16 | 16 | 16 | 16 | 19 | 21 | 14 | 16 | 15 | 19 | 20 | 23 | 23 | 31 | 30 | 13 | 16 | 16 | 29 | 29 | 29 | 29 | 30 | 31 | 31 |
| 2 | FT4m(5,35,50) | 13 | 15 | 15 | 17 | 15 | 14 | 18 | 16 | 11 | 10 | 11 | 10 | 11 | 10 | 11 | 10 | 10 | 10 | 10 | 11 | 11 | 13 | 13 | 17 | 18 | 12 |
| 3 | FT5(5,35) | 4 | 7 | 6 | 10 | 9 | 7 | 23 | 14 | 4 | 3 | 4 | 3 | 4 | 3 | 4 | 3 | 3 | 3 | 3 | 4 | 4 | 6 | 6 | 11 | 13 | 6 |
| 3 | FT5(5,35) | 3 | 7 | 6 | 10 | 9 | 7 | 23 | 14 | 4 | 3 | 4 | 3 | 4 | 3 | 4 | 3 | 3 | 3 | 3 | 4 | 4 | 6 | 6 | 11 | 13 | 6 |
| Index1 | Index < Threshold: given + | | + | + | + | + | + | + | + | + | + | + | + | - | - | - | - | + | + | + | - | - | - | - | - | - | - |
| Index2 | Index > Threshold: given + | | + | + | + | + | + | + | + | - | - | - | - | - | - | - | - | - | - | - | - | - | + | + | + | + | - |
| Index3 | Index < Threshold H: given + | | - | - | - | - | - | - | - | + | + | + | + | + | + | + | + | + | + | + | - | - | - | - | - | - | - |
| Index3 | Index < Threshold L: given + | | - | - | - | - | - | - | - | - | - | - | - | - | - | - | - | + | + | + | - | - | - | - | - | - | - |
| | Determination | | LA | LA | LA | LA | LA | LA | LA | Def | Def | Def | Def | Def | Def | Def | Def | In3 | In3 | In3 | In2 | In2 | In1 | In1 | In1 | In1 | In2 |

2) Combination of 9:1 sample mixture and 1:9 sample mixture

Sample mixtures with mixing ratios (test sample:NP) of 9:1 and 1:9 (9:1 sample mixture and 1:9 sample mixture, respectively) were prepared, and their respective Tm(X) was calculated. The following indexes 1 and 2 were calculated for the test samples (LA, Def, and Inh).

Index 1; FT1(5) of the 9:1 sample mixture
Index 2; FT6(95)

$$[FT1(X) = [(Tm(X) - Tn(X)) / (Ts(X) - Tn(X))] \times 100$$

FT6(X) = [Tm(X) of the 9:1 sample mixture - Tm(X) of the 1:9 sample mixture]]

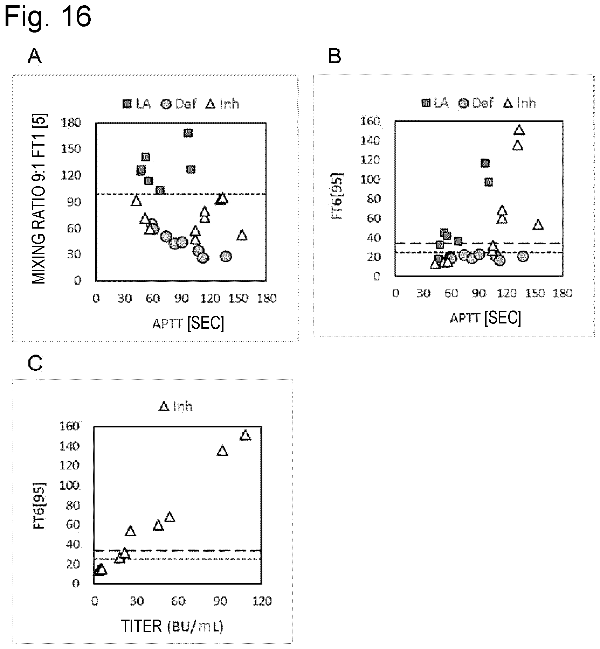

A mean and standard deviation (SD) of Def(D-1 to D-8) was determined for each of the indexes 1 and 2. Threshold: mean + 4SD was used for the index 1. Threshold L: the mean + 2SD and threshold H: the mean + 6SD, were used for the index 2. Figures 16A and 16B show the calculated indexes 1 and 2 of each test sample, and Figure 16C shows a plot of the index 2 of Inh against a titer. In Figure 16A, the dotted line depicts the threshold. In Figures 16B and 16C, the dotted line depicts the threshold L, and the broken line depicts the threshold H.
The indexes 1 and 2 were compared with the threshold, and a sample species of the test sample was estimated according to the following criteria on the basis of the comparison results.

<Estimation criteria>

(i) Index 1 > threshold: "LA"
The remaining samples were further divided in the following order.
(ii) Index 2 < threshold L: "Def"
(iii) Index 2 > threshold H: "Inh (In1)"
(iv) Index 2 ≤ threshold H: "Inh (In2)"

Table 8 shows the indexes 1 and 2 of each test sample, results of comparing these indexes with the threshold, and the estimated sample species. The present estimation criteria enabled LA to be discriminated from the other sample species (Def and Inh) and also enabled Def to be accurately identified. As for Inh, high-titer Inh having a titer of 46 or more (In1; I-6 to I-9) and middle-titer Inh having a titer of 18 or more and less than 46 (In2; I-4, I-5) were able to be identified. On the other hand, low-titer Inh having a titer of 5 or less (I-1 to I-3) was discriminated from Def.

[Table 8]

| Sample species and ID No. thereof | | LA | | | | | | | Def | | | | | | | | Inh | | | | | | | | | |
|---|---|---|---|---|---|---|---|---|---|---|---|---|---|---|---|---|---|---|---|---|---|---|---|---|---|---|
| | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | | - | - | - | - | - | - | - | - | - | - | - | - | - | - | - | 3 | 5 | 5 | 18 | 22 | 46 | 54 | 92 | 108 | 26 |
| APTT [sec] | | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | FT1(5) with a mixing ratio of 9:1 | 99 | 125 | 127 | 140 | 113 | 103 | 168 | 127 | 65 | 59 | 51 | 43 | 44 | 35 | 27 | 28 | 92 | 72 | 59 | 48 | 58 | 73 | 79 | 94 | 95 | 53 |
| 2 | FT6 (95) | 25 | 18 | 33 | 45 | 42 | 37 | 117 | 97 | 21 | 19 | 23 | 19 | 24 | 22 | 17 | 22 | 14 | 15 | 15 | 27 | 32 | 60 | 69 | 136 | 152 | 54 |
| 2 | FT6 (95) | 34 | 18 | 33 | 45 | 42 | 37 | 117 | 97 | 21 | 19 | 23 | 19 | 24 | 22 | 17 | 22 | 14 | 15 | 15 | 27 | 32 | 60 | 69 | 136 | 152 | 54 |
| Index1 | Index > Threshold: given + | | + | + | + | + | + | + | + | - | - | - | - | - | - | - | - | - | - | - | - | - | - | - | - | - | - |
| Index2 | Index < Threshold L: given + | | + | - | - | - | - | - | - | + | + | + | + | + | + | + | + | + | + | + | - | - | - | - | - | - | - |
| Index2 | Index > Threshold H: given + | | - | - | + | + | + | + | + | - | - | - | - | - | - | - | - | - | - | - | - | - | + | + | + | + | + |
| | Determination | | LA | LA | LA | LA | LA | LA | LA | Def | Def | Def | Def | Def | Def | Def | Def | Def | Def | Def | In2 | In2 | In1 | In1 | In1 | In1 | In1 |

## Example 6

Indexes 1 to 4 given below were calculated for the test samples (LA, Def, and Inh). Tm(X) was calculated using sample mixtures with a mixing ratio (test sample:NP) of 1:1.

Index 1 = FT1(45)
Index 2 = FT2(5H)
Index 3 = FT2(45)
Index 4 = FT2(45)-FT2(5)

$$[FT1(X) = (Tm(X) - Tn(X)) / (Ts(X) - Tn(X)) \times 100$$

$$FT2(X) = (Tm(X) / Tn(X)) \times 100]$$

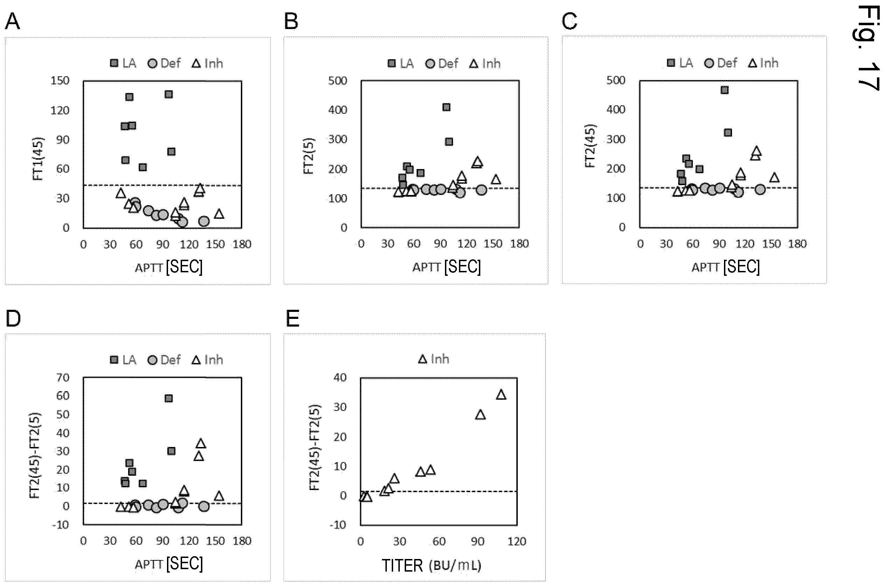

A mean and standard deviation (SD) of Def(D-1 to D-8) was determined for each of the indexes 1 to 4. The mean + 4SD was used as a threshold for the index 1, and the mean + 1SD was used for the indexes 2 to 4. Figures 17A to 17D show the calculated indexes 1 to 4 of each test sample, and Figure 17E shows a plot of the index 4 of Inh against a titer. In each diagram, the dotted line depicts the threshold.
The indexes 1 to 4 were compared with the threshold, and a sample species of the test sample was estimated according to the following criteria on the basis of the comparison results.

<Estimation criteria>

(i) Index 1 > threshold: "LA"
The remaining samples were further divided in the following order.
(ii) Index 2 < threshold and index 3 < threshold: "Def"
(iii) Index 4 > threshold: "Inh"

Table 9 shows the indexes 1 to 4 of each test sample, results of comparing these indexes with the threshold, and the estimated sample species. The present estimation criteria enabled LA to be discriminated from the other sample species (Def and Inh) and also enabled Inh having a titer exceeding 5 to be accurately identified. On the other hand, low-titer Inh having a titer of 5 or less (I-1 to I-3) was discriminated from Def.

[Table 9]

| Sample species and ID No. thereof | | LA | | | | | | | Def | | | | | | | | Inh | | | | | | | | | |
|---|---|---|---|---|---|---|---|---|---|---|---|---|---|---|---|---|---|---|---|---|---|---|---|---|---|---|
| | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | | - | - | - | - | - | - | - | - | - | - | - | - | - | - | - | 3 | 5 | 5 | 18 | 22 | 46 | 54 | 92 | 108 | 26 |
| APTT [sec] | | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | FT1(45) | 43 | 104 | 69 | 133 | 104 | 62 | 136 | 78 | 27 | 22 | 19 | 14 | 14 | 10 | 7 | 7 | 36 | 25 | 21 | 13 | 16 | 24 | 26 | 38 | 41 | 15 |
| 2 | FT2(5) | 134 | 169 | 146 | 210 | 198 | 186 | 410 | 293 | 132 | 129 | 133 | 130 | 133 | 132 | 120 | 130 | 124 | 125 | 126 | 136 | 145 | 169 | 178 | 219 | 227 | 166 |
| 3 | FT2(45) | 134 | 183 | 158 | 233 | 217 | 198 | 469 | 323 | 133 | 129 | 134 | 129 | 134 | 132 | 122 | 130 | 124 | 125 | 125 | 138 | 147 | 177 | 187 | 247 | 261 | 172 |
| 4 | FT2(45)-FT2(5) | 1 | 14 | 13 | 24 | 19 | 13 | 59 | 30 | 1 | 0 | 1 | -1 | 1 | -1 | 2 | 0 | 0 | 0 | 0 | 2 | 2 | 8 | 9 | 28 | 34 | 6 |
| Index1 | Index > Threshold: given + | | + | + | + | + | + | + | + | - | - | - | - | - | - | - | - | - | - | - | - | - | - | - | - | - | - |
| Index2 | Index < Threshold: given + | | - | - | - | - | - | - | - | + | + | + | + | + | + | + | + | + | + | + | - | - | - | - | - | - | - |
| Index3 | Index < Threshold: given + | | - | - | - | - | - | - | - | + | + | + | + | + | + | + | + | + | + | + | - | - | - | - | - | - | - |
| Index4 | Index > Threshold: given + | | + | + | + | + | + | + | + | - | - | - | - | - | - | - | - | - | - | - | + | + | + | + | + | + | + |
| | Determination | | LA | LA | LA | LA | LA | LA | LA | Def | Def | Def | Def | Def | Def | Def | Def | Def | Def | Def | Inh | Inh | Inh | Inh | Inh | Inh | Inh |

Comparative Example

The discrimination of LA, Def, and Inh was attempted using conventional APTT-based indexes Percent Correction (PC) and Rosner Index (RI).

1) 1:1 sample mixture

Indexes 1 and 2 given below were calculated for the test samples (LA, Def, and Inh) with APTT as T(50). Tm(X) was calculated using sample mixtures with a mixing ratio (test sample:NP) of 1:1.

Index 1; PC(%) = (Ts(50) - Tm(50)) / (Ts(50) - Tn(50)) $\times$ 100

$$\text{Index 2; RI(\%) = (Tm(50) - Tn(50)) / Ts(50)} \times 100$$

A mean - 4SD of Def(D-1 to D-8) was set to a threshold for the index 1. A mean + 2SD of Def(D-1 to D-8) was set to a threshold for the index 2.

Figures 18A and 18B show the indexes 1 and 2 of the test samples, and Figures 18C and 18D show plots of the indexes 1 and 2 of Inh against a titer. In each diagram, the dotted line depicts the threshold. Both the indexes 1 and 2 exhibited a different distribution of LA from that of Def or Inh, whereas the distributions of Def and Inh overlapped with each other (Figures 18A and 18B). As for Inh, the index 1 was distributed in a mountain shape peaking at a titer of 18, and the index 2 was distributed in a J shape with a titer of 18 as the lowest point (Figures 18C and 18D).

The indexes 1 and 2 of each test sample were compared with the threshold, and a sample species of the test sample was estimated according to the following criteria on the basis of the comparison results.

<Estimation criteria>

(i) Index 1 < threshold: "LA"
The remaining samples were further divided in the following order.
(ii) Index 2 $\leq$ threshold: "Def"
(iii) Index 2 > threshold: "Inh"

Table 10 shows the indexes 1 and 2 of each test sample, results of comparing these indexes with the threshold, and the estimated sample species. The present estimation criteria enabled LA to be discriminated from the other sample species (Def and Inh). However, a high-titer sample of Inh having a titer of 46 or more was able to be identified, whereas Inh having a titer of less than 46 was not able to be discriminated from Def.

[Table 10]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | PC | 57 | -4 | 30 | -32 | -3 | 39 | -35 | 22 | 73 | 78 | 82 | 87 | 86 | 90 | 93 | 93 | 65 | 76 | 79 | 88 | 85 | 77 | 74 | 64 | 60 | 85 |
| 2 | RI | 16 | 47 | 32 | 67 | 55 | 38 | 99 | 58 | 15 | 12 | 12 | 9 | 10 | 8 | 5 | 6 | 14 | 12 | 11 | 9 | 12 | 17 | 20 | 29 | 32 | 12 |
| Index1 | Index < Threshold: given + | | + | + | + | + | + | + | + | - | - | - | - | - | - | - | - | - | - | - | - | - | - | - | - | - | - |
| Index2 | Index > Threshold: given + | | + | + | + | + | + | + | + | - | - | - | - | - | - | - | - | - | - | - | - | - | + | + | + | + | - |
| | | Determination | LA | LA | LA | LA | LA | LA | LA | Def | Def | Def | Def | Def | Def | Def | Def | Def | Def | Def | Def | Def | Inh | Inh | Inh | Inh | Def |

2) 9:1 sample mixture

The indexes 1 and 2 calculated in 1) described above were calculated using sample mixtures with a mixing ratio (test sample:NP) of 9:1. A mean - 3.5SD of Def(D-1 to D-8) was set to a threshold for the index 1. A mean + 1.5SD of Def(D-1 to D-8) was set to a threshold for the index 2.

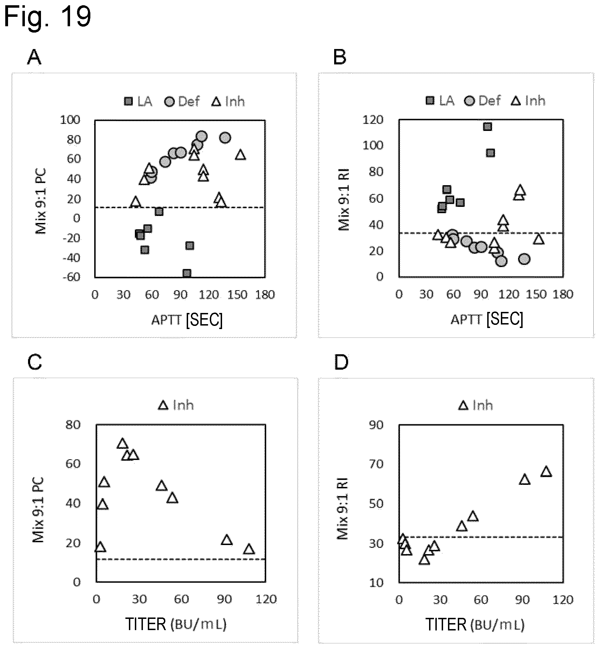

Figures 19A and 19B show the indexes 1 and 2 of the test samples, and Figures 19C and 19D show plots of the indexes 1 and 2 of Inh against a titer. In each diagram, the dotted line depicts the threshold. The index 1 of LA was distributed beneath Def or Inh, whereas the distributions of Def and Inh overlapped with each other (Figure 19A). As for the index 2, the distributions of LA and Inh or Def and Inh overlapped with each other (Figure 19B). As for Inh, the index 1 was distributed in a mountain shape peaking at a titer of 18, and the index 2 was distributed in a J shape with a titer of 18 as the lowest point (Figures 19C and 19D).

The indexes 1 and 2 of each test sample were compared with the threshold, and a sample species of the test sample was estimated according to the estimation criteria described above in 1) on the basis of the comparison results. Table 11 shows the indexes 1 and 2 of each test sample, results of comparing these indexes with the threshold, and the estimated sample species. The present estimation criteria enabled LA to be discriminated from the other sample species (Def and Inh). However, a high-titer sample of Inh having a titer of 46 or more was able to be identified, whereas Inh having a titer of less than 46 was not able to be discriminated from Def, as in the 1:1 sample mixtures (Table 10).

[Table 11]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | PC | 12 | -16 | -18 | -32 | -10 | 7 | -56 | -28 | 42 | 48 | 58 | 66 | 67 | 75 | 84 | 83 | 18 | 40 | 51 | 71 | 64 | 49 | 43 | 22 | 17 | 65 |
| 2 | RI | 33 | 52 | 54 | 67 | 59 | 57 | 115 | 95 | 32 | 29 | 28 | 23 | 23 | 19 | 13 | 14 | 33 | 30 | 27 | 22 | 27 | 39 | 44 | 63 | 67 | 29 |
| Index1 | Index < Threshold: given + | | + | + | + | + | + | + | + | - | - | - | - | - | - | - | - | - | - | - | - | - | - | - | - | - | - |
| Index2 | Index > Threshold: given + | | + | + | + | + | + | + | + | - | - | - | - | - | - | - | - | - | - | - | - | - | + | + | + | + | - |
| | | Determination | LA | LA | LA | LA | LA | LA | LA | Def | Def | Def | Def | Def | Def | Def | Def | Def | Def | Def | Def | Def | Inh | Inh | Inh | Inh | Def |

3) 1:9 sample mixture

The indexes 1 and 2 calculated in 1) described above were calculated using sample mixtures with a mixing ratio (test sample:NP) of 1:9. A mean - 4SD of Def(D-1 to D-8) was set to a threshold for the index 1. A mean + 2.5SD of Def(D-1 to D-8) was set to a threshold for the index 2.

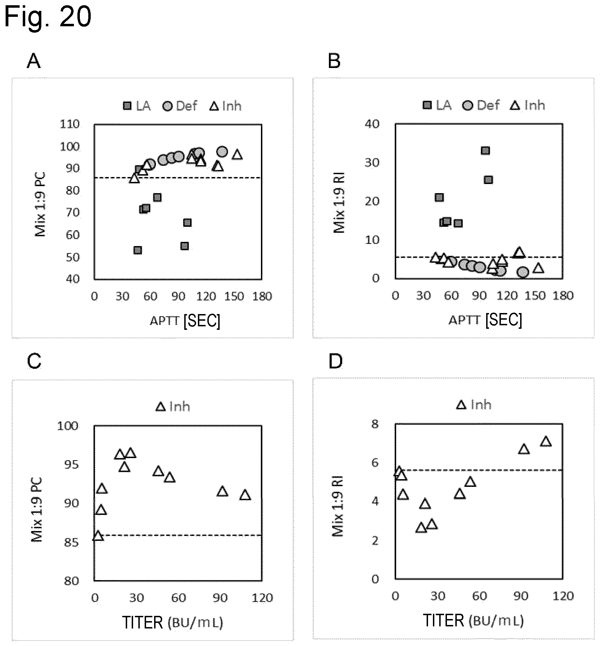

Figures 20A and 20B show the indexes 1 and 2 of the test samples, and Figures 20C and 20D show plots of the indexes 1 and 2 of Inh against a titer. In each diagram, the dotted line depicts the threshold. Both the indexes 1 and 2 exhibited a different distribution of LA from that of Def or Inh, whereas the distributions of Def and Inh overlapped with each other (Figures 20A and 20B). As for Inh, the index 1 was distributed in a mountain shape peaking at a titer of 26, and the index 2 was distributed in a J shape with a titer of 18 as the lowest point (Figures 20C and 20D).

The indexes 1 and 2 of each test sample were compared with the threshold, and a sample species of the test sample was estimated according to the estimation criteria described above in 1) on the basis of the comparison results.

Table 12 shows the indexes 1 and 2 of each test sample, results of comparing these indexes with the threshold, and the estimated sample species. The present estimation criteria caused only one sample of LA (L-2) to be misestimated as Def and however, enabled the other samples of LA to be discriminated from Def and Inh. As for Inh, only a high-titer sample having a titer of 92 or more was able to be identified, and Inh having a titer of less than 92 was not able to be discriminated from Def.

[Table 12]

| Sample species and ID No. thereof | LA | | | | | | | Def | | | | | | | | Inh | | | | | | | | | |
|---|---|---|---|---|---|---|---|---|---|---|---|---|---|---|---|---|---|---|---|---|---|---|---|---|---|
| | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| Inhibitor titer [BU/mL] | - | - | - | - | - | - | - | - | - | - | - | - | - | - | - | 3 | 5 | 5 | 18 | 22 | 46 | 54 | 92 | 108 | 26 |
| APTT [sec] | 47 | 48 | 53 | 56 | 68 | 97 | 100 | 59 | 60 | 74 | 83 | 90 | 108 | 112 | 137 | 43 | 52 | 57 | 105 | 105 | 115 | 115 | 132 | 133 | 154 |

| Index | Index symbol | Threshold | LA | | | | | | | Def | | | | | | | | Inh | | | | | | | | | |
|---|---|---|---|---|---|---|---|---|---|---|---|---|---|---|---|---|---|---|---|---|---|---|---|---|---|---|---|
| | | | L-1 | L-2 | L-3 | L-4 | L-5 | L-6 | L-7 | D-1 | D-2 | D-3 | D-4 | D-5 | D-6 | D-7 | D-8 | I-1 | I-2 | I-3 | I-4 | I-5 | I-6 | I-7 | I-8 | I-9 | I-10 |
| 1 | PC | 86 | 53 | 90 | 71 | 72 | 77 | 55 | 66 | 92 | 92 | 94 | 95 | 96 | 97 | 97 | 98 | 86 | 89 | 92 | 96 | 95 | 94 | 93 | 92 | 91 | 97 |
| 2 | RI | 5.9 | 21 | 5 | 14 | 15 | 14 | 33 | 25 | 5 | 5 | 4 | 4 | 3 | 2 | 2 | 2 | 6 | 5 | 4 | 3 | 4 | 4 | 5 | 7 | 7 | 3 |
| Index1 | Index < Threshold: given + | | + | - | + | + | + | + | + | - | - | - | - | - | - | - | - | - | - | - | - | - | - | - | - | - | - |
| Index2 | Index > Threshold: given + | | + | - | + | + | + | + | + | - | - | - | - | - | - | - | - | - | - | - | - | - | - | - | + | + | - |
| | Determination | | LA | Def | LA | LA | LA | LA | LA | Def | Def | Def | Def | Def | Def | Def | Def | Def | Def | Def | Def | Def | Def | Def | Inh | Inh | Def |

**Claims**

1. A coagulation time extension factor estimation method, comprising:

   1) obtaining Tm(X) and Tn(X); and
   2) estimating a coagulation time extension factor for sample S on the basis of Tm(X),

   wherein

   Tm(X) represents a measurement point or a time at which a coagulation reaction curve of sample M reaches X% of coagulation reaction end point Em,
   Tn(X) represents a measurement point or a time at which a coagulation reaction curve of sample N reaches X% of coagulation reaction end point En,
   Em is a coagulation reaction end point in the coagulation reaction curve of sample M,
   En is a coagulation reaction end point in the coagulation reaction curve of sample N,
   the sample S is a test blood sample having an extended coagulation time,
   the sample N is a normal blood sample,
   the sample M is a sample mixture of the sample S and the sample N, and
   X is larger than 0 and 100 or smaller.

2. The method according to claim 1, wherein

   the 1) further comprises detecting coagulation reaction end point Es in a coagulation reaction curve of the sample S and obtaining Ts(X) on the basis of Es, wherein
   Ts(X) represents a measurement point or a time at which the coagulation reaction curve of the sample S reaches X% of Es.

3. The method according to claim 2, wherein
   the 2) comprises:

   (i) calculating one or more of indexes Rj,
   wherein

$$j \geq 1,$$

   each of the indexes Rj is calculated on the basis of at least one member selected from the group consisting of Ts(X), Tm(X), and Tn(X), and
   at least one of the indexes Rj is calculated on the basis of at least Tm(X);

   (ii) comparing each of the indexes Rj with threshold Tj; and
   (iii) estimating the coagulation time extension factor for the sample S on the basis of the comparison result, wherein

   the coagulation time extension factor is lupus anticoagulant positivity, coagulation factor deficiency, or coagulation factor inhibitor positivity.

4. The method according to claim 2, wherein
the 2) comprises:

calculating at least one index selected from the group consisting of the following R1 to R8:

R1: an index which reflects a ratio between a difference between Tm(X) and Tn(X) and a difference between Ts(X) and Tn(X) for specific X,
R2: an index which represents an amount of change in R1(X) in a specific range of X,
R3: an index which reflects a ratio between an amount of change in Tm(X) and an amount of change in Tn(X) in a specific range of X,
R4: an index which reflects a relative rate of change in T(X) in a specific range of X for the sample S or the sample M,
R5: an index which reflects an amount of change in Tm(X) in a specific range of X,
R6: an index which reflects a difference in Tm(X) between the samples M differing in a mixing ratio of the sample S and the sample N,
R7: an index which reflects a ratio between Tm(X) and Tn(X) for specific X, and
R8: an index which represents an amount of change in R7(X) in a specific range of X; and

estimating the coagulation time extension factor for the sample S using the calculated index.

5. The method according to claim 2, wherein
the 2) comprises:

calculating the following index:

$$R1(Xa) = [(Tm(Xa) - Tn(Xa)) / (Ts(Xa) - Tn(Xa))]$$

wherein Xa = from 5 to 100; and
estimating the sample S to have lupus anticoagulant positivity when R1(Xa) is higher than threshold T1, or in other cases, estimating the sample S to have coagulation factor deficiency or coagulation factor inhibitor positivity.

6. The method according to claim 2, wherein
the 2) comprises:

calculating the following indexes:

$$R4s(Xx,Xy,Xz) = [(Ts(Xx) - Ts(Xy)) / Ts(Xz)]$$

$$R4m(Xx,Xy,Xz) = [(Tm(Xx) - Tm(Xy)) / Tm(Xz)]$$

wherein Xx = from 5 to 30, Xy = from 10 to 95, and Xz = from 5 to 95, or Xx = from 45 to 70, Xy = from 10 to 45, and Xz = from 5 to 95, and Xx # Xy; and
setting threshold T4s for R4s and threshold T4m for R4m, and estimating the sample S to have lupus anticoagulant positivity when R4s of the sample S is lower than the threshold T4s and R4m is higher than the threshold T4m.

7. The method according to claim 5 or 6, wherein
the method comprises:

calculating, for the sample S which has not been estimated to have lupus anticoagulant positivity, the following index:

$$R2(Xb,Xc) = [R1(Xb) - R1(Xc)],$$

wherein Xb = from 5 to 55, Xc = from 10 to 95, and Xb # Xc; and
estimating the sample S to have coagulation factor inhibitor positivity when R2(Xb,Xc) is higher than threshold T2, or estimating the sample S to have coagulation factor deficiency when R2(Xb,Xc) is lower than threshold T2.

8. The method according to claim 7, wherein
   the method comprises:

   calculating, for the sample S estimated to have coagulation factor deficiency, the following index:

   $$R3(Xd,Xe) = [Tm(Xd) - Tm(Xe)] / [Tn(Xd) - Tn(Xe)]$$

   wherein Xd = from 5 to 90, Xe = from 10 to 95, and Xe - Xd $\geq$ 5; and
   estimating the sample S to have coagulation factor inhibitor positivity when R3(Xd,Xe) is lower than threshold T3.

9. The method according to claim 7, wherein
   the method comprises:

   as for the sample S estimated to have coagulation factor deficiency, estimating the sample S to have coagulation factor inhibitor positivity when index R3(Xd,Xe) is lower than threshold T3L; and
   as for the sample S estimated to have coagulation factor inhibitor positivity, estimating the sample S to be a coagulation factor inhibitor-positive sample with a high inhibitor titer when R3(Xd,Xe) is higher than threshold T3H, or as for the sample S estimated to have coagulation factor inhibitor positivity, estimating the sample S to be a coagulation factor inhibitor-positive sample with an intermediate inhibitor titer when R3(Xd,Xe) is between thresholds T3L and T3H,
   wherein

   $$R3(Xd,Xe) = [Tm(Xd) - Tm(Xe)] / [Tn(Xd) - Tn(Xe)]$$

   wherein Xd = from 5 to 90, Xe = from 10 to 95, Xe - Xd $\geq$ 5, and

   $$T3L < T3H.$$

10. The method according to claim 7, wherein
    the method comprises:
    calculating, for the sample S estimated to have coagulation factor inhibitor positivity, the following index:

    R1(Xa') = [(Tm(Xa') - Tn(Xa')) / (Ts(Xa') - Tn(Xa'))] wherein Xa' = from 5 to 80; and
    estimating the sample S to be a coagulation factor inhibitor-positive sample with a high inhibitor titer when R1(Xa') is higher than threshold T1'.

11. The method according to claim 5 or 6, wherein
    the method comprises:

    calculating, for the sample S which has not been estimated to have lupus anticoagulant positivity, the following index:

    $$R5(Xb,Xc) = [Tm(Xc) - Tm(Xb)]$$

    wherein Xb = from 5 to 35, Xc = 20 to 95, and Xb < Xc; and
    estimating the sample S to have coagulation factor deficiency when R5(Xb,Xc) is higher than threshold T5L and lower than threshold T5H (wherein T5L < T5H), or in other cases, estimating the sample S to have coagulation factor inhibitor positivity.

12. The method according to claim 11, wherein the method comprises, as for the sample S estimated to have coagulation factor inhibitor positivity, estimating the sample S to be a coagulation factor inhibitor-positive sample with a low inhibitor titer when R5(Xb,Xc) is lower than the threshold T5L, or as for the sample S estimated to have coagulation factor inhibitor positivity, estimating the sample S to be a coagulation factor inhibitor-positive sample with a relatively high inhibitor titer when R5(Xb,Xc) is higher than the threshold T5H.

13. The method according to claim 5 or 6, wherein
    the method comprises:

calculating, for the sample S which has not been estimated to have lupus anticoagulant positivity, the following indexes:

$$R7(Xb) = [Tm(Xb) / Tn(Xb)]$$

$$R7(Xb') = [Tm(Xb') / Tn(Xb')]$$

$$R8(Xb,Xb') = [R7(Xb) - R7(Xb')]$$

wherein Xb = from 5 to 95, Xb' = from 5 to 95, and Xb # Xb'; and
estimating the sample S to have coagulation factor deficiency when R7(Xb) is lower than threshold T7 and R7(Xb') is lower than threshold T7', and estimating the sample S to have coagulation factor inhibitor positivity when R8(Xb,Xb') is higher than threshold T8.

14. The method according to claim 5 or 6, further comprising the following:

obtaining $Tm_A(X)$ and $Tm_B(X)$ for the sample S which has not been estimated to have lupus anticoagulant positivity,
wherein $Tm_A(X)$ represents a measurement point or a time at which a coagulation reaction curve of sample mixture A reaches X% of coagulation reaction end point Em, $Tm_B(X)$ represents a measurement point or a time at which a coagulation reaction curve of sample mixture B reaches X% of coagulation reaction end point Em, the sample mixture A is a sample mixture having a volume ratio of the sample S and the sample N of 9:1, the sample mixture B is a sample mixture having a volume ratio of the sample S and the sample N of 1:9, and X is larger than 0 and 100 or smaller;
calculating the following index:

$$R6(Xb) = [Tm_A(Xb) - Tm_B(Xb)]$$

wherein Xb = from 35 to 95; and
estimating the sample S to have coagulation factor deficiency when R6(Xb) is lower than threshold T6, or estimating the sample S to have coagulation factor inhibitor positivity when R6(Xb) is higher than threshold T6.

15. The method according to claim 14, wherein the method comprises, as for the sample S estimated to have coagulation factor inhibitor positivity, estimating the sample S to be a coagulation factor inhibitor-positive sample with a high inhibitor titer when R6(Xb) is higher than threshold T6'.

16. A program for carrying out a method according to claim 1 or 2.

17. An apparatus for carrying out a method according to claim 1 or 2.

Fig. 1

Fig. 2

Fig. 3

Fig. 4

A
[AMOUNT OF SCATTERED LIGHT]

B
[AMOUNT OF SCATTERED LIGHT]

C
[AMOUNT OF SCATTERED LIGHT]

EP 4 506 692 A1

# Fig. 5

Fig. 6

A

[AMOUNT OF SCATTERED LIGHT]

--- NP   —··— LA   —··· Def   —— Inh

B

[AMOUNT OF SCATTERED LIGHT]

--- NP   —··— LA-M11   —··· Def-M11   —— Inh-M11

# Fig. 7

# Fig. 8

A    MIXING RATIO 1:9

B    MIXING RATIO 1:1

C    MIXING RATIO 9:1

# Fig. 9

A   MIXING RATIO 1:9

FT1(X)-FT1(95)

B   MIXING RATIO 1:1

FT1(X)-FT1(95)

C   MIXING RATIO 9:1

FT1(X)-FT1(95)

## Fig. 10

Fig. 11

Fig. 12

A

B

Fig. 13

A

B

C

Fig. 14

Fig. 15

A

B

C

D

Fig. 16

# Fig. 17

# Fig. 18

Fig. 19

# Fig. 20

# Fig. 21

| | |
|---|---|
| MEASURE COAGULATION REACTION OF TEST SAMPLE | s1 |
| OBTAIN REACTION CURVE Ps(i) OF TEST SAMPLE | s2 |
| CALCULATE Ts(X) FROM Ps(i) | s3 |
| MEASURE COAGULATION REACTION OF SAMPLE MIXTURE | s4 |
| OBTAIN REACTION CURVE Pm(i) OF SAMPLE MIXTURE | s5 |
| CALCULATE Tm(X) FROM Pm(i) | s6 |
| CALCULATE INDEX Rj FROM Ts(X), Tm(X), AND Tn(X) | s7 |
| COMPARE INDEX Rj WITH THRESHOLD Tj | s8 |
| ESTIMATE EXTENSION FACTOR FROM COMPARISON RESULTS | s9 |
| OUTPUT COAGULATION TIME AND EXTENSION FACTOR | s10 |

# Fig. 22

## INTERNATIONAL SEARCH REPORT

| International application No. |
|---|
| **PCT/JP2023/011644** |

### A. CLASSIFICATION OF SUBJECT MATTER

***G01N 33/86*** (2006.01)i
FI:   G01N33/86

According to International Patent Classification (IPC) or to both national classification and IPC

### B. FIELDS SEARCHED

Minimum documentation searched (classification system followed by classification symbols)

G01N33/86

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2019-086517 A (SEKISUI MEDICAL CO LTD) 06 June 2019 (2019-06-06) paragraphs [0012]-[0028], fig. 1-6 | 16-17 |
| A | WO 2016/136558 A1 (SCHOOL JURIDICAL PERSON HIGASHI-NIPPON-GAKUEN) 01 September 2016 (2016-09-01) entire text, all drawings | 1-17 |
| A | WO 2016/152305 A1 (HITACHI HIGH-TECHNOLOGIES CORPORATION) 29 September 2016 (2016-09-29) entire text, all drawings | 1-17 |
| A | WO 2009/153964 A1 (SEKISUI MEDICAL CO LTD) 23 December 2009 (2009-12-23) entire text, all drawings | 1-17 |
| A | JP 2016-194426 A (SCHOOL JURIDICAL PERSON HIGASHI-NIPPON-GAKUEN) 17 November 2016 (2016-11-17) entire text, all drawings | 1-17 |
| A | WO 2021/132552 A1 (SEKISUI MEDICAL CO LTD) 01 July 2021 (2021-07-01) entire text, all drawings | 1-17 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 June 2023** | **13 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/011644**

| Patent document cited in search report | | | Publication date (day/month/year) | Patent family member(s) | | | Publication date (day/month/year) |
|---|---|---|---|---|---|---|---|
| JP | 2019-086517 | A | 06 June 2019 | (Family: none) | | | |
| WO | 2016/136558 | A1 | 01 September 2016 | US entire text, all drawings EP CN | 2017/0350907 3264096 107250801 | A1 A1 A | |
| WO | 2016/152305 | A1 | 29 September 2016 | US entire text, all drawings EP CN | 2018/0080948 3273250 107430139 | A1 A1 A | |
| WO | 2009/153964 | A1 | 23 December 2009 | US entire text, all drawings EP CN KR | 2011/0129862 2290370 102066947 10-2011-0027661 | A1 A1 A A | |
| JP | 2016-194426 | A | 17 November 2016 | US entire text, all drawings EP CN | 2016/0291042 3076173 106018282 | A1 A1 A | |
| WO | 2021/132552 | A1 | 01 July 2021 | EP entire text, all drawings CN | 4083630 114829943 | A1 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6696963 B
- JP 6871673 B
- JP 6249855 A
- JP 2019237427 A
- JP 2020039344 A
- JP 2020068877 A
- JP 2020048676 W